# EUROPÄISCHE PATENTANMELDUNG

(11) **EP 3 876 193 A1**
(43) Veröffentlichungstag der Anmeldung: **08.09.2021**
(21) Anmeldenummer: 20160329.7
(22) Anmeldetag: 02.03.2020
(51) Int. Cl.: G06T 7/00

(54) **BILDVERARBEITUNGSVERFAHREN ZUM ANZEIGEN VON ZELLEN MEHRERER GESAMTBILDER**

(71) Anmelder: Euroimmun Medizinische Labordiagnostika AG, 23560 Lübeck (DE)
(72) Erfinder: Gerlach, Stefan, 23627 Groß Grönau (DE); Marzahl, Christian, 91052 Erlangen (DE); Voigt, Jörn, 23558 Lübeck (DE); Kröger, Christine, 23623 Ahrensbök (DE)

(57) **Zusammenfassung**

Vorgeschlagen wird ein Bildverarbeitungsverfahren zum Anzeigen von Zellen mehreren Pathologiebildern bzw. Gesamtbildern. Ein jeweiliges Gesamtbild repräsentiert eine jeweilige Patientengewebeprobe oder eine jeweilige Patientenzellprobe. Das Verfahren weist die Schritte auf: Bereitstellen der Gesamtbilder, Detektieren von einzelnen Zellbildern in den Gesamtbildern mittels eines rechnergestützten Algorithmus, Bestimmen von Klassifikationsdaten mittels des rechnergestützten Algorithmus, wobei die Klassifikationsdaten eine jeweilige eindeutige Zuordnung eines jeweiligen detektierten Zellbildes zu einer von mehreren Zellklassen indizieren, und wobei die Klassifikationsdaten ferner ein jeweiliges Konfidenzmaß bezogen auf die jeweilige eindeutige Zuordnung aufweisen, Generieren von jeweiligen Klassenbildern für die jeweiligen Zellklassen, wobei ein Klassenbild einer Zellklasse die der Zellklasse zugeordneten Zellbilder in einer regelmäßigen Anordnung und mit einer vorbestimmten Abfolge widergibt und wobei ferner die Abfolge der zugeordneten Zellbilder in Abhängigkeit der Konfidenzmaße der zugeordneten Zellbilder gewählt wird, sowie ferner Anzeigen eines Teilausschnittes wenigstens eines Klassenbildes.

## Beschreibung

Die Erfindung betrifft ein Bildverarbeitungsverfahren zum Anzeigen von Zellen mehrerer Gesamtbilder. Die Erfindung betrifft ferner ein Bildverarbeitungsverfahren zur Durchführung auf einem Server, sowie ein Bildverarbeitungsverfahren zur Durchführung auf einem Client, als auch entsprechende Computerprogrammprodukte zur Ausführung auf einem Computer zur Durchführung der Verfahren auf dem Server bzw. dem Client.

Im Zuge pathologischer Untersuchungen ist es eine wesentliche Aufgabe eines Pathologen, mehrere Gesamtbilder zu analysieren, wobei ein Gesamtbild eine Patientengewebeprobe in Form eines Gewebeschnittes repräsentiert oder wobei ein Gesamtbild eine Patientenzellprobe in Form eines Zellausstrichs einer flüssigen Patientenprobe repräsentiert. Eine derartige Analyse von Gesamtbildern um Zuge einer pathologische Untersuchung erfolgt üblicherweise in der Art, dass der Pathologe in zumindest mehreren Teilbereichen eines jeden Gesamtbildes die dargestellten einzelnen Zellen bzw. Zellbilder unterschiedliche Zellklassen zuordnet werden und dann für jede Zellklasse die Anzahl der in der Klasse vorkommenden Zellen zählt. Auf Basis der jeweiligen Zellanzahl einer jeweiligen Zellklasse erstellt der Pathologe dann einen Befund. Hierbei wird üblicherweise im Zuge einer Untersuchung nicht nur ein einzelnes Gesamtbild in der zuvor beschriebenen Weise durch den Pathologen analysiert, sondern mehrere Gesamtbilder, welche auf Patientenproben eines gleichen Patienten zurückzuführen sind. Für eine Befundung auf Basis von Gesamtbildern, welche als Gewebeschnitten ist es beispielsweise üblich, dass ein Pathologe je Gesamtbild mehrere Teilbereiche als sogenannte Hauptgesichtsfelder ("High-Power Field") hinsichtlich vorliegender Zellen der Zellklasse Mitosezelle analysiert, wobei ein Hauptgesichtsfeld eine Teilbereichsfläche von 0,1 bis 0,4 mm2 des Gesamtbildes wiedergibt und wobei üblicherweise 50 Hauptgesichtsfelder eines Gesamtbildes bzw. eines Gewebeschnittes analysiert werden.

Ein einzelnes Gesamtbild weist eine sehr große Menge an einzelnen Zellbildern auf, sodass selbst bei der Möglichkeit einer starken optischen Vergrößerung des Gesamtbildes durch ein Mikroskop oder beispielsweise durch eine digitale Anzeigeeinheit wie einen Computermonitor zur Gewinnung eines Hauptgesichtsfeldes der Pathologe eine sehr große Anzahl von Zellen unterschiedlicher Zellklassen gleichzeitig betrachten muss, um die Zellen den Zellklassen zuzuordnen und für jede Zellklasse die jeweilige Zellanzahl zu bestimmen. Betrachtet ein Pathologe einen bestimmten Bildausschnitt eines Gesamtbildes, so sieht er hierbei jeweilige Zellen unterschiedlicher Zellklassen an jeweiligen räumlichen Positionen, wie sie sich aufgrund der räumlichen Struktur der Probe ergeben.

Im Zuge einer pathologischen Untersuchung eines oder mehrerer Gesamtbilder ist es ferner üblich, dass eine derartige Untersuchung bzw. Befundung nicht nur durch einen einzigen Pathologen erfolgt, sondern auch noch einmal durch einen weiteren, zweiten Pathologen überprüft und gegebenenfalls korrigiert wird. Zellklassenzuordnungen, die ein erster Pathologe vorgenommen hat, können dann gegebenenfalls durch den zweiten Pathologen geändert bzw. korrigiert werden.

Die Gesamtbilder repräsentieren entweder jeweils eine Patientengewebeprobe in Form eines Gewebeschnittes oder die Gesamtbilder repräsentieren jeweils eine Patientenzellprobe in Form eines Zellausstrichs einer flüssigen Patientenprobe. Für Gesamtbilder, welche jeweils eine Patientengewebeprobe in Form eines Gewebeschnittes repräsentieren, ist eine erste Zellklasse die Klasse Mitosezelle und eine zweite Klasse die Klasse Nicht-Mitosezelle. Für Gesamtbilder, welche jeweils eine Patientenzellprobe in Form eines Zellausstrichs einer flüssigen Patientenprobe repräsentieren, ist eine erste Zellklasse die Klasse Makrophagen, eine zweite Zellklasse die Klasse Lymohozyten, eine dritte Zellklasse die Klasse Eosinophile.

In dem Fall, dass die Gesamtbilder jeweils eine Patientengewebeprobe in Form eines Gewebeschnittes repräsentieren, sind wenigstens zwei Zellklassen gegeben aufweisend als eine erste Zellklasse die Klasse Mitosezelle und als eine zweite Klasse die Klasse Nicht-Mitosezelle. In dem Fall, dass die Gesamtbilder jeweils eine Patientenzellprobe in Form eines Zellausstrichs einer flüssigen Patientenprobe repräsentieren, sind wenigstens zwei Zellklassen gegeben ausgewählt aus der Gruppe die Klasse Makrophagen, die Klasse Lymohozyten und die Klasse Eosinophile.

Aufgabe der Erfindung ist es, ein Bildverarbeitungsverfahren zum Anzeigen von Zellen mehrerer Gesamtbilder bereitzustellen, bei welcher in rechnergestützter Weise ein Klassifikationsergebnis bzgl. einzelner Zellbilder und eine Zellzählung für jeweilige Zellklassen für die mehreren Gesamtbilder durch den Pathologen besonders zuverlässig überprüft werden kann.

Die erfindungsgemäße Aufgabe wird durch das vorgeschlagene Bildverarbeitungsverfahren zum Anzeigen von Zellen mehrerer Gesamtbilder gelöst.

Ein jeweiliges Gesamtbild repräsentiert hierbei eine jeweilige Patientengewebeprobe oder eine jeweilige Patientenzellprobe, insbesondere ein Bild eines Zellausstrichs einer flüssigen Patientenzellprobe.

Zunächst werden die Gesamtbilder bereitgestellt. Es erfolgt dann mittels eines rechnergestützten Algorithmus, beispielsweise eines Neural Networks, ein Detektieren von einzelnen Zellbildern in den Gesamtbildern. Ferner erfolgt mittels des computergestützten Algorithmus eine Bestimmung von Klassifikationsdaten. Die Klassifikationsdaten indizieren eine jeweilige eindeutige Zuordnung eines jeweiligen detektierten Zellbildes zu einer von mehreren Zellklassen. Ferner weisen die Klassifikationsdaten ein jeweiliges Konfidenzmaß bezogen auf die jeweilige eindeutige Zuordnung eines detektierten Zellbildes zu einer der mehreren Zellklassen auf. Zellen einer gleichen Klasse werden also einem gleichen Klassenbild zugeordnet.

Es erfolgt ferner ein Generieren von jeweiligen Klassenbildern für die jeweiligen Zellklassen. Hierbei gibt ein Klassenbild einer Zellklasse die der Zellklasse zugeordneten Zellbilder in einer regelmäßigen Anordnung und mit einer vorbestimmten Abfolge wieder. Die vorbestimmte Abfolge wird in Abhängigkeit der Konfidenzmaße der zugeordneten Zellbilder des Klassenbildes bzw. der entsprechenden Zellklasse gewählt. Vorzugsweise beginnt die Abfolge mit dem Zellbild, welches das höchste Konfidenzmaß aufweist, und es folgend dann weitere Zellbilder entsprechend abnehmender Konfidenzmaße. Schließlich erfolgt ein Anzeigen wenigstens eines Teilausschnittes wenigstens eines Klassenbildes. Vorzugsweise kann ein Nutzer durch eine Nutzereingabe eine Zuordnung eines Zellbildes zu einer Zellklasse bzw. dem entsprechenden Klassenbild modifizieren und so korrigieren, vorzugsweise auf dem Client. Vorzugsweise werden mehrere Teilausschnitte mehrerer Klassenbilder zeitlich nacheinander bzw. zeitlich sequentiell angezeigt.

Das erfindungsgemäße vorgeschlagene Bildverarbeitungsverfahren birgt unterschiedliche mögliche Vorteile, welche nun im Weiteren genauer erläutert werden.

Mittels des computergestützten Algorithmus werden einzelne Zellbilder in den Gesamtbildern detektiert und dann unterschiedlichen Zellklassen zugeordnet. Würde lediglich ein Gesamtbild einer Gewebeprobe oder Zellprobe mit optischen Markierungen entsprechend der Zellklassen versehen werden und dann angezeigt werden, wobei die jeweiligen räumlichen Anordnungen der jeweiligen einzelnen Zellbilder unverändert bleiben würden, so wäre es dem Pathologen prinzipiell möglich, kann dies zu einer visuellen Gesamtinformation führen, welche für einen Nutzer bzw. Pathologen schwer erfassbar sein kann.

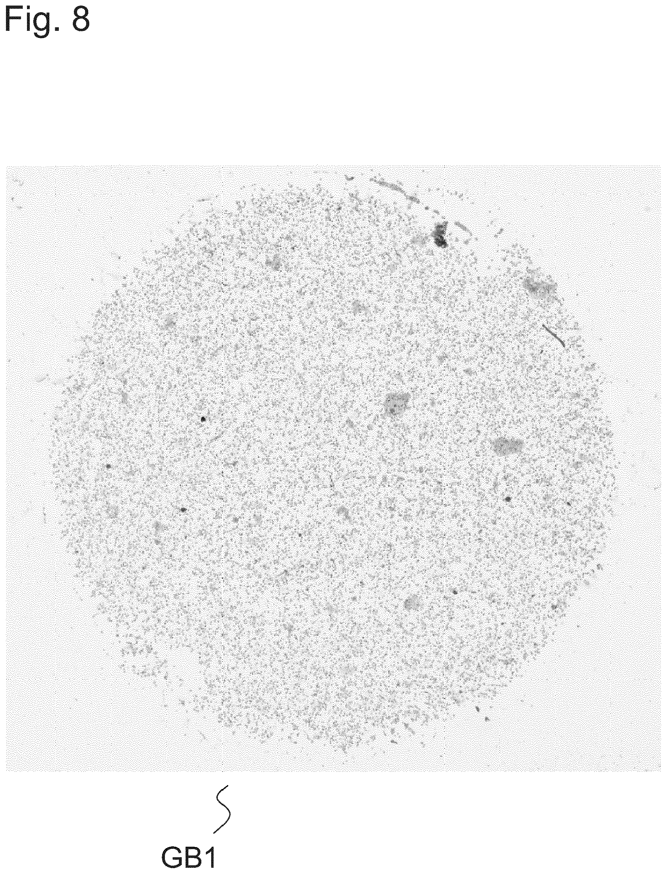

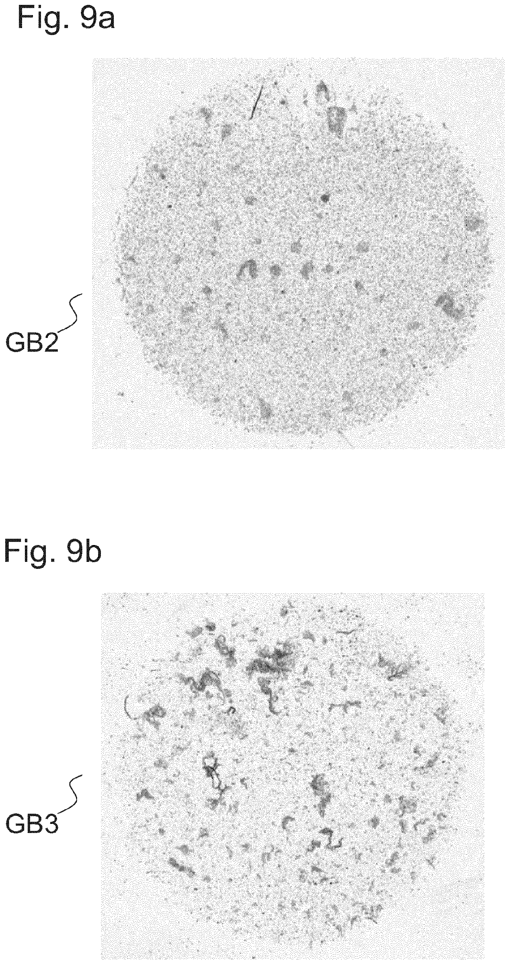

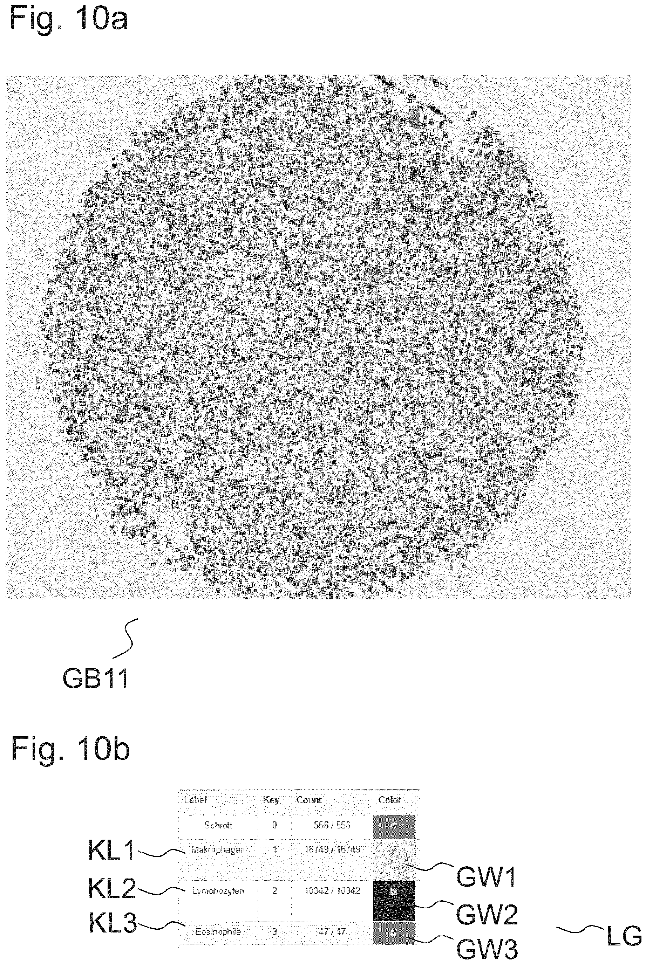

Fig. 8 zeigt hierzu beispielhaft ein Gesamtbild GB1, in diesem Fall einer Patientenzellprobe in Form eines Zellausstrichs. Weitere beispielhafte Patientenzellproben in Form von Zellausstrichen sind in den Fig. 9A und 9B als Gesamtbilder GB2 bzw. GB3 dargestellt. Die Fig. 10A zeigt ein Klassifikationsergebnis der Patientenzellprobe aus Fig. 8 als ein Gesamtbild GB11, bei welchem kleine Rechtecke unterschiedlicher Grautöne für unterschiedliche Zellklassen jeweils die Klassifikationsergebnisse einzelner detektierter Zellen bzw. einzelner detektierter Zellbilder indizieren. Die Fig. 10B zeigt hierzu beispielhafte Zellklassen KL1, KL2, KL3 wie beispielsweise Makrophagen, Lymohozyten oder Eosinophile, welche als Rechtecke in dem Bild GB11 mit entsprechenden Grauwerten GW1, GW2, GW3 dargestellt sind. Bei dieser Darstellungsweise nach dem Stand der Technik ergibt sich eine große Menge an visueller Gesamtinformation für einen Pathologen. Theoretisch ist es zwar möglich, das Gesamtbild GB11 optisch sehr stark zu vergrößern, so dass dann der Pathologe dieses Gesamtbild GB11 abschnittsweise betrachten könnte, um die Klassifikationsergebnisse des rechnergestützten Algorithmus zu überprüfen und ggf. zu korrigieren. Hierzu müsste dann aber eben der Pathologe bei Betrachtung eines entsprechenden vergrößerten Abschnittes gleichzeitig Zellen unterschiedlicher Zellklassen betrachten und überprüfen muss, wobei die räumliche Anordnung der Zellen abhängig von der Struktur der Probe wäre.

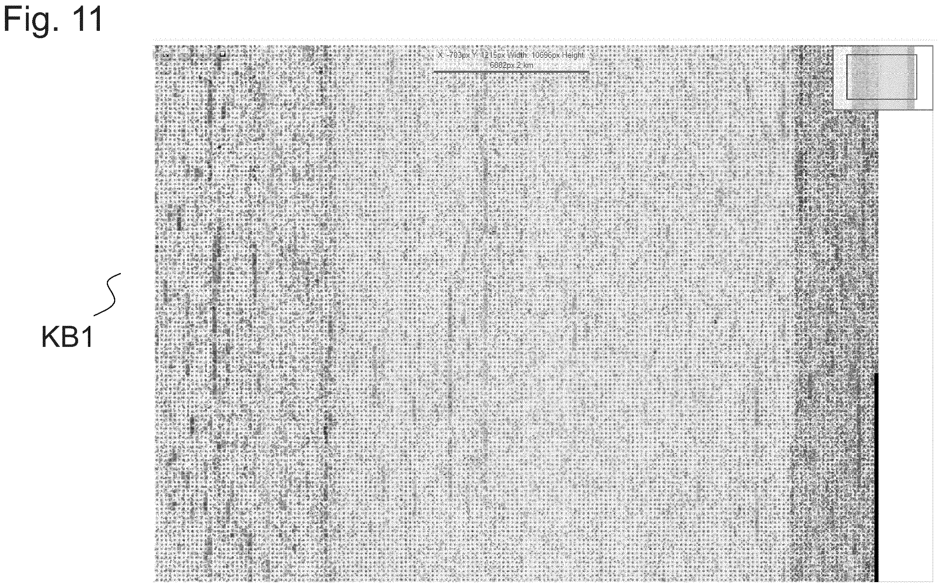

Die Fig. 11 zeigt ein erfindungsgemäßes Klassenbild KB1, welches detektierte einzelne Zellbilder aus den mehreren Gesamtbildern GB1, GB2, GB3 für eine bestimmte einzelne Zellklasse zeigt, welche zu dem Klassenbild korrespondiert. Mit anderen Worten, in dem Klassenbild KB1 sind nur solche einzelne Zellbilder in regelmäßiger Anordnung bzw. regelmäßiger räumlicher Anordnung dargestellt, welche zu einer einzelnen Zellklasse gehören. Ferner wird die Abfolge der zugeordneten Zellbilder des Klassenbildes KB1 in Abhängigkeit der Konfidenzmaße der zugeordneten Zellbilder gewählt. Beispielsweise kann in der linken oberen Ecke des Klassenbildes KB1 jenes Zellbild angeordnet sein, für welches der computergestützte Algorithmus das höchste Konfidenzmaß bezüglich der Zuordnung dieses einzelnen Zellbildes zu dem Klassenbild KB1 bzw. der zugehörigen Zellklasse ermittelt hat. Die Zellbilder können dann beispielsweise bezüglich ihrer Abfolge mit absteigendem Konfidenzmaß in der obersten Zeile von links nach rechts gehend angeordnet werden, wobei dann in der nächstfolgenden zweiten Zeile die räumliche regelmäßige Anordnung fortgeführt wird. Ein Klassenbild KB1 entspricht mit anderen Worten einer Matrix als eine regelmäßige räumliche Anordnung von einzelnen Zellbildern.

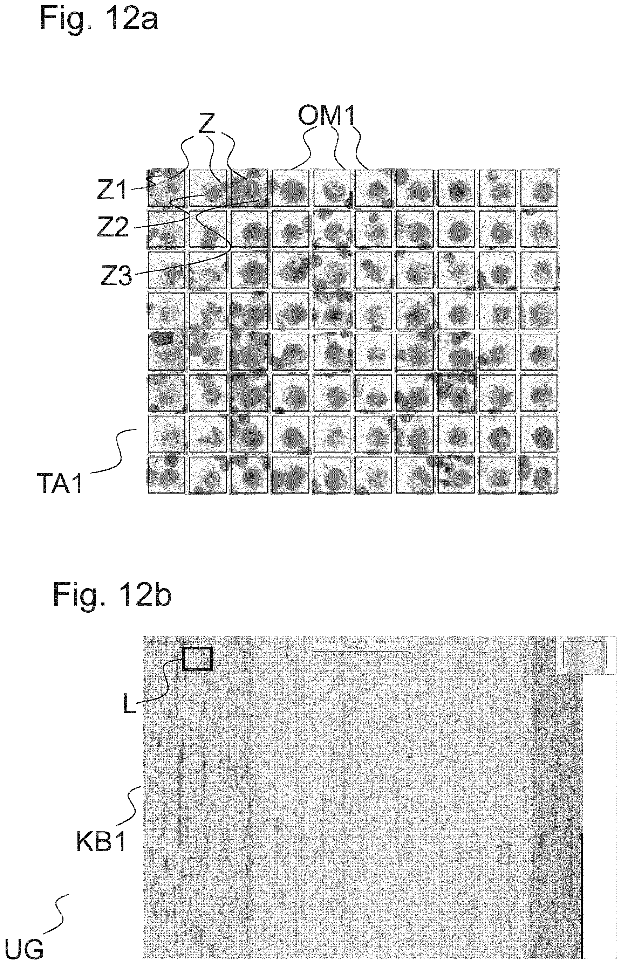

Die Fig. 12A zeigt einen beispielhaften Teilausschnitt TA1 mit 10x8 einzelnen detektierten Zellbildern Z, Z1, Z2, Z3 eines gleichen Klassenbildes bzw. einer gleichen Zellklasse. Der Pathologen kann dann diesen Teilausschnitt eines Klassenbildes betrachten und durch visuelle Inspektion die einzelnen Zellbilder Z hinsichtlich ihrer Zuordnung zu dem entsprechenden Klassenbild bzw. der entsprechenden Zellklasse überprüfen. Es wird also nicht das gesamte Klassenbild sondern nur ein Teilausschnitt hieraus angezeigt, so dass ein zu verwendendes Anzeigemittel die Zellbilder Z des Teilausschnittes in hinreichender Vergrößerung darstellen kann.

Der Vorteil des erfindungsgemäßen Verfahrens liegt darin, dass der Pathologe nun bei Betrachtung des Teilausschnitte TA1 des Klassenbildes KB1 nur solche einzelnen Zellbilder dargestellt bekommt, welche zu einer gleichen Zellklasse durch das computergestützte Verfahren zugeordnet wurden. Ferner stellt die erfindungsgemäß gewählte Abfolge der einzelnen Zellbilder in dem Klassenbild KB1 für den Pathologen inhärent eine Information dar, für welche einzelnen Zellbilder der Algorithmus ein hohes Konfidenzmaß bezogen auf die jeweilige Zuordnung des Zellbilder zu der Zellklasse ermittelt hat. Der Pathologe kann dann also in der oberen linken Ecke mit der Betrachtung der einzelnen Zellbilder beginnen, um die hier dargestellte Zuordnung der Zellbilder zu der Zellklasse des Klassenbildes KB1 zu überprüfen. Der Pathologe muss nicht, wie in der Fig. 10A zu sehen, mehrere einzelne Zellbilder unterschiedlicher Klassen in zufälliger räumlicher Anordnung gleichzeitig betrachten und dann durch visuelle Inspektion überprüfen, ob die durch den computergestützten Algorithmus vorgenommene Zuordnung von Zellen zur jeweiligen Zellklasse korrekt ist. Er kann bei Betrachtung des Teilausschnittes TA1 sich hierbei auf eine einzelne Zellklasse konzentrieren. Er kann ferner aufgrund der Position eines Zellbildes Z in dem Klassenbild bzw. dem Teilausschnitt TA1 erfassen, wie sicher sich der rechnergestützte Algorithmus bei der Zuordnung eines Zellbildes zu der entsprechenden Klasse war, da Zellbilder mit einem hohen Konfidenzmaß sich in anderen Bildregionen befinden als Zellbilder mit niedrigen Konfidenzmaßen. Daher kann der Pathologe aus der Position eines Zellbildes darauf schließen, wie sehr er der Zuordnung eines Zellbildes zu der entsprechenden Zellklasse durch den rechnergestützten Algorithmus vertrauen kann.

Der Pathologe kann also als ein Nutzer angesehen werden, welcher die technische Aufgabe des Überprüfens einer Zuordnung von Zellbildern zu Zellklassen aufgrund der erfindungsgemäßen Lösung besonders effizient und schnell durchführen kann. Dieser Aspekt einer effizienten und schnellen Durchführung einer Aufgabe durch einen Nutzer bei einem Anzeigen von Bildern kann in Analogie zu dem Verfahren zum Anzeigen von Bildern aus der Entscheidung des europäischen Patentamtes T0642/00, siehe europäische Patentanmeldung Nr. 93300749.4, gesehen werden.

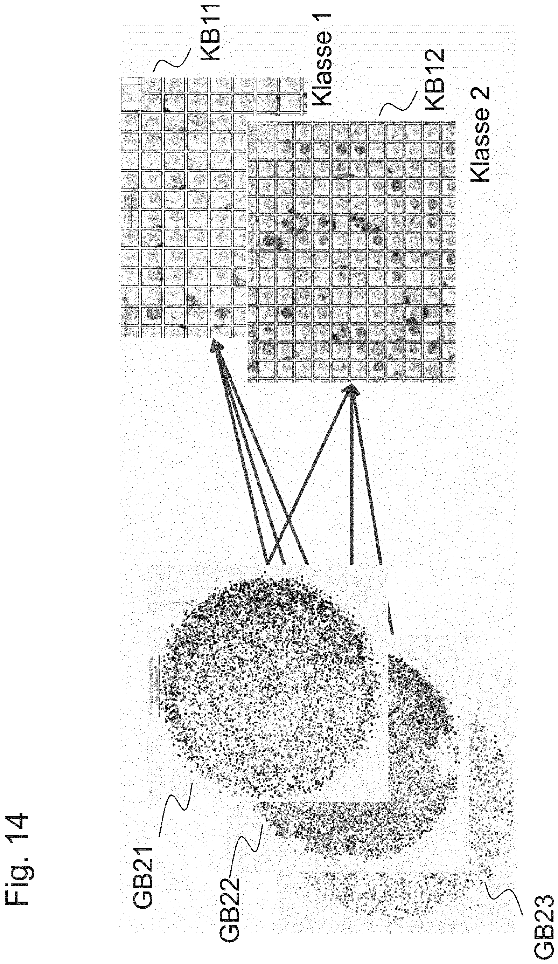

Die Fig.14 illustriert noch einmal auf andere Weise einen möglichen Vorteil des vorgeschlagenen Bildverarbeitungsverfahrens. Aus den mehreren Gesamtbildern GB11, GB12, GB 13 können einzelne Zellbilder detektiert und klassifiziert werden, hier zum Beispiel in zwei unterschiedliche Klassen Klasse1 und Klasse2. Für jede der Zellenklassen wird dann ein entsprechendes Klassenbild KB11, KB12 generiert. Es wird dann ein Teilausschnitt eines Klassenbildes angezeigt. Der Pathologe kann dann also bei Betrachten eines Teilausschnittes daraus mehrere Zellbilder aus mehreren Gesamtbildern in Bezug auf die Zuordnung der Zellbilder zu einer bestimmten Zellklasse gemeinsam und gleichzeitig betrachten. Ferner kann bei einem Anzeigen von Teilausschnitten unterschiedlicher Klassenbilder KB11, KB12, vorzugsweise zeitlich sequenziell nacheinander, der Pathologe dann Zellbilder unterschiedlicher Zellklassen nacheinander überprüfen, wobei er aber in einem einzelnen Teilausschnitt immer nur Zellbilder einer einzelnen Klasse überprüfen muss.

Vorteilhafte Ausführungsformen der Erfindung sind Gegenstand der abhängigen Ansprüche und werden in der folgenden Beschreibung unter teilweiser Bezugnahme auf die Figur näher erläutert.

Vorzugsweise erfolgen die zuvor genannten Schritte des Bereitstellens, des Detektierens, des Bestimmens und des Generierens auf einem Server. Das Verfahren weist ferner vorzugsweise folgende Schritte auf: Generieren, auf dem Server, eines Annotationsdatensatzes, welcher für jedes der Klassenbilder jeweilige Zuordnungen der jeweiligen Zellbilder zu einer zu dem Klassenbild korrespondierenden Zellklasse indiziert und welcher ferner jeweilige örtliche Positionen der jeweiligen Zellbilder innerhalb der Klassenbilder indiziert, Anfordern eines Teilausschnittes eines Klassenbildes durch einen Client, Übertragen des Teilausschnittes sowie ferner wenigstens eines zu dem Teilausschnitt korrespondierenden Teil-Annotationsdatensatzes von dem Server an den Client und temporäres Abspeichern des Teil-Annotationsdatensatzes auf dem Client, Generieren, auf dem Client, von jeweiligen optischen Markierungen für jeweilige Zellbilder des Teilausschnittes auf Basis wenigstens des Teil-Annotationsdatensatzes, wobei die jeweiligen optischen Markierungen jeweilige Zuordnungen der jeweiligen Zellbilder zu einer zu dem Klassenbild korrespondierenden Zellklasse indizieren, sowie Anzeigen des Teilausschnittes sowie der zugehörigen optischen Markierungen auf dem Client auf einer Anzeigeeinheit.

Vorzugsweise weist das Verfahren ferner die Schritte auf: Entgegennehmen, auf dem Client, einer Nutzereingabe, welche eine Auswahl eines angezeigten Zellbildes indiziert und welche ferner eine neue Zuordnung des ausgewählten Zellbildes zu einer modifizierten Zellklasse indiziert, Generieren und Anzeigen, auf dem Client, einer neuen optischen Markierung für das ausgewählte Zellbild korrespondierend zu der modifizierten Zellklasse, Modifizieren des temporär abgespeicherten Teil-Annotationsdatensatzes in Korrespondenz zu dem ausgewählten Zellbild und der modifizierten Zellklasse auf dem Client, Übermitteln einer Information von dem Client an den Server, welche das ausgewählte Zellbild und die modifizierte Zellklasse indiziert, sowie Modifizieren des Annotationsdatensatzes auf dem Server in Korrespondenz zu dem ausgewählten Zellbild und der modifizierten Zellklasse.

Vorzugsweise ist der Teilausschnitt ein erster Teilausschnitt. Vorzugsweise weist das Verfahren ferner die Schritte auf: Anfordern eines zweiten Teilausschnittes des Klassenbildes durch den Client, Übertragen des zweiten Teilausschnittes sowie ferner wenigstens eines zu dem zweiten Teilausschnitt korrespondierenden zweiten Teil-Annotationsdatensatzes von dem Server an den Client und temporäres Abspeichern des zweiten Teil-Annotationsdatensatzes auf dem Client, Generieren, auf dem Client, von jeweiligen optischen Markierungen für jeweilige Zellbilder des zweiten Teilausschnittes auf Basis wenigstens des zweiten Teil-Annotationsdatensatzes, wobei die jeweiligen optischen Markierungen jeweilige Zuordnungen der jeweiligen Zellbilder zu einer zu dem Klassenbild korrespondierenden Zellklasse indizieren, sowie Anzeigen des zweiten Teilausschnittes sowie zugehöriger optischen Markierungen auf dem Client.

Vorzugsweise weist das Verfahren ferner die Schritte auf: Entgegennehmen einer Synchronisationsanforderung sowie Synchronisieren der Klassifikationsdaten mit dem modifizierten Annotationsdatensatz auf dem Server.

Vorgeschlagen wird ferner ein Bildverarbeitungsverfahren zur Durchführung auf einem Server, aufweisend die Schritte: Bereitstellen von mehreren Gesamtbildern, wobei ein jeweiliges Gesamtbild eine jeweilige Patientengewebeprobe oder eine jeweilige Patientenzellprobe repräsentiert, Detektieren von einzelnen Zellbildern in den Gesamtbildern mittels eines rechnergestützten Algorithmus, Bestimmen von Klassifikationsdaten mittels des rechnergestützten Algorithmus, wobei die Klassifikationsdaten eine jeweilige eindeutige Zuordnung eines jeweiligen detektierten Zellbildes zu einer jeweiligen Zellklasse indizieren, und wobei die Klassifikationsdaten ferner ein jeweiliges Konfidenzmaß bezogen auf die jeweilige eindeutige Zuordnung aufweisen, Generieren von jeweiligen Klassenbildern für die jeweiligen Zellklassen, wobei ein Klassenbild einer Zellklasse die der Zellklasse zugeordneten Zellbilder in einer regelmäßigen Anordnung und mit einer vorbestimmten Abfolge der zugeordneten Zellbilder in Abhängigkeit der Konfidenzmaße der zugeordneten Zellbilder gewählt wird, Generieren eines Annotationsdatensatzes, welcher für jedes der Klassenbilder jeweilige Zuordnungen der jeweiligen Zellbilder zu einer zu dem Klassenbild korrespondierenden Zellklasse indiziert und welcher ferner jeweilige örtliche Positionen der jeweiligen Zellbilder innerhalb der Klassenbilder indiziert, Übertragen eines Teilausschnittes wenigstens eines Klassenbildes sowie ferner wenigstens eines zu dem Teilausschnitt korrespondierenden Teil-Annotationsdatensatzes an einen Client, Entgegennehmen einer Information von dem Client, welche ein ausgewähltes Zellbild und eine modifizierte Zellklasse indiziert, sowie Modifizieren des Annotationsdatensatzes in Korrespondenz zu dem ausgewählten Zellbild und der modifizierten Zellklasse.

Das Bildverarbeitungsverfahren zur Durchführung auf einem Server weist ferner vorzugsweise die Schritte auf: Entgegennehmen einer Synchronisationsanforderung sowie Synchronisieren der Klassifikationsdaten mit dem modifizierten Annotationsdatensatz.

Vorgeschlagen wird ferner ein Bildverarbeitungsverfahren zur Durchführung auf einem Client, aufweisend die Schritte: Anfordern eines Teilausschnittes wenigstens eines Klassenbildes von einem Server, wobei ein Klassenbild zu einer Zellklasse zugeordnete Zellbilder in einer regelmäßigen Anordnung und mit einer vorbestimmten Abfolge widergibt, Empfangen des Teilausschnittes des Klassenbildes sowie ferner wenigstens eines zu dem Teilausschnitt korrespondierenden Teil-Annotationsdatensatzes von dem Server und temporäres Abspeichern des Teil-Annotationsdatensatzes, wobei der Teil-Annotationsdatensatz für den Teilausschnitt des Klassenbildes jeweilige Zuordnungen jeweiliger Zellbilder zu einer zu dem Klassenbild korrespondierenden Zellklasse indiziert und wobei der Teil-Annotationsdatensatz jeweilige örtliche Positionen der jeweiligen Zellbilder innerhalb des Klassenbildes indiziert, Generieren von jeweiligen optischen Markierungen für jeweilige Zellbilder des Teilausschnittes auf Basis wenigstens des Teil-Annotationsdatensatzes, wobei die jeweiligen optischen Markierungen jeweilige Zuordnungen der jeweiligen Zellbilder zu einer zu dem Klassenbild korrespondierenden Zellklasse indizieren, sowie Anzeigen des Teilausschnittes sowie zugehöriger optischer Markierungen von Zellbildern des Teilausschnittes.

Das Bildverarbeitungsverfahren zur Durchführung auf einem Client weist ferner vorzugsweise die Schritte auf: Entgegennehmen einer Nutzereingabe, welche eine Auswahl eines angezeigten Zellbildes indiziert und welche ferner eine neue Zuordnung des ausgewählten Zellbildes zu einer modifizierten Zellklasse indiziert, Generieren und Anzeigen einer neuen optischen Markierung für das ausgewählte Zellbild korrespondierend zu der modifizierten zugeordneten Zellklasse, Modifizieren des temporär abgespeicherten Teil-Annotationsdatensatzes in Korrespondenz zu dem ausgewählten Zellbild und der modifizierten Zellklasse, sowie Übermitteln einer Information an das Server, welche das ausgewählte Zellbild und die modifizierte Zellklasse indiziert.

Vorgeschlagen wird ferner ein Computerprogrammprodukt umfassend Befehle, die bei der Ausführung des Programms durch einen Computer in Form eines Servers diesen veranlassen, das Bildverarbeitungsverfahren zur Durchführung auf einem Server durchzuführen.

Vorgeschlagen wird ferner ein Computerprogrammprodukt umfassend Befehle, die bei der Ausführung des Programms durch einen Computer in Form eines Clients diesen veranlassen, das Bildverarbeitungsverfahren zur Durchführung auf einem Client durchzuführen.

Im Folgenden wird die Erfindung anhand spezieller Ausführungsformen ohne Beschränkung des allgemeinen Erfindungsgedankens anhand der Figuren näher erläutert. Dabei zeigen:
Fig. 1 Schritte zur Durchführung des erfindungsgemäßen Bildverarbeitungsverfahrens zum Anzeigen von Zellen mehrerer Gesamtbilder in einem Teilausschnitt eines Klassenbildes,
Fig. 2 beispielhafte Konfidenzmaßdaten,
Fig. 3 ein beispielhaftes Klassenbild,
Fig. 4 bevorzugte Schritte zur Durchführung des Bildverarbeitungsverfahrens,
Fig. 5 weitere bevorzugte Schritte zur Durchführung des Bildverarbeitungsverfahrens,
Fig. 6 wiederum weitere bevorzugte Schritte zur Durchführung des Bildverarbeitungsverfahrens,
Fig. 7A einen Server,
Fig. 7B einen Client,
Fig. 8, 9A sowie 9B beispielhafte Gesamtbilder,
Fig. 10A das Gesamtbild aus Fig. 8 gemeinsam mit optischen Markierungen einzelner Zellbilder,
Fig. 10B eine Legende für unterschiedliche Grautöne optischer Markierungen aus der Figur 10A bezogen auf unterschiedliche Zellklassen,
Fig. 11 ein beispielhaftes Klassenbild,
Fig. 12A ein Teilausschnitt eines Klassenbildes,
Fig. 12B eine beispielhafte Lage eines Teilausschnittes innerhalb des Klassenbildes aus Fig. 11,
Fig. 13A einen anderen Teilausschnitt einer anderen Zellklasse bzw. eines anderen Klassenbildes gemeinsam mit entsprechenden optischen Markierungen,
Fig. 13B den Teilausschnitt aus Fig.12A sowie eine modifizierte optische Markierung eines einzelnen Zellbildes,
Figur 14 mehrere Gesamtbilder mit mehreren Klassenbildern.

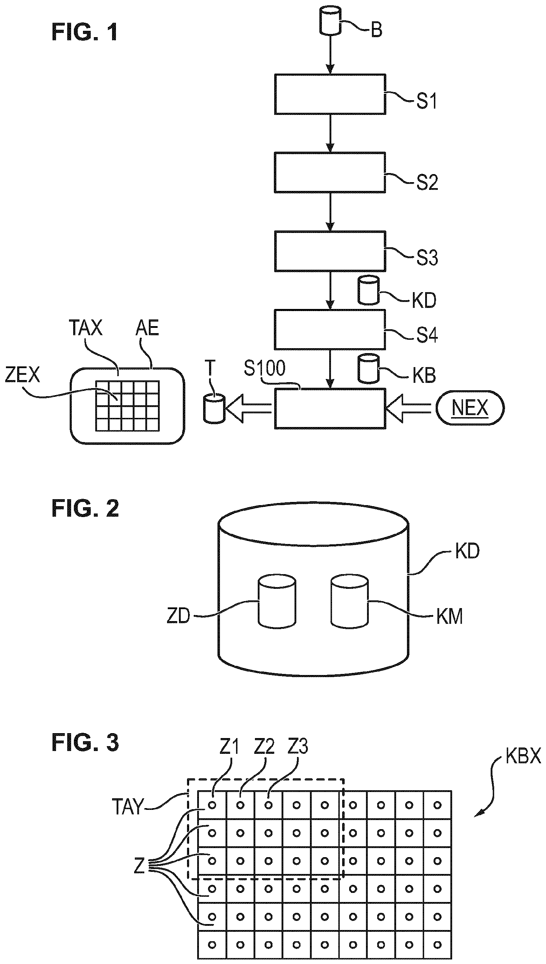

Die Fig. 1 illustriert eine bevorzugte Abfolge von Schritten zur Durchführung des erfindungsgemäßen Bildverarbeitungsverfahrens. In einem Schritt S1 erfolgt ein Bereitstellen der Gesamtbilder als Bilddaten B, beispielsweise der Gesamtbilder GB1, GB2, GB3 aus den Fig. 8, 9A sowie 9B. In einem Schritt S2 erfolgt ein Detektieren von einzelnen Zellbildern in den Gesamtbildern mittels eines computergestützten Algorithmus wie beispielsweise eines Neural Network. In einem Schritt S3 erfolgt ein Bestimmen von Klassifikationsdaten KD mittels des computergestützten Algorithmus. Die Klassifikationsdaten KD indizieren eine jeweilige eindeutige Zuordnung eines jeweiligen detektierten Zellbildes zu einer von mehreren Zellklassen. Die Klassifikationsdaten KD weisen ferner für ein jeweiliges detektiertes Zellbild ein jeweiliges Konfidenzmaß bezogen auf die jeweilige eindeutige Zuordnung des detektierten Zellbildes zu einer der mehreren Zellklassen auf.

In einem Schritt S4 erfolgt ein Generieren von jeweiligen Klassenbildern bzw. Klassenbilddaten KB für die jeweiligen Zellklassen, wobei ein Klassenbild einer Zellklasse die der Zellklasse zugeordneten Zellbilder in einer regelmäßigen Anordnung mit einer vorbestimmten Abfolge wiedergibt. Ein Beispiel eines Klassenbildes ist das Klassenbild KB1 aus der Fig. 11.

In einem Schritt S100 erfolgt dann ein Anzeigen eines Teilausschnittes TAX eines Klassenbildes, beispielsweise des Teilausschnittes TA1 der Fig. 12A. Es wird also ein Teilausschnitt TAX in Form von Teilausschnittsdaten T an eine Anzeigeeinheit AE, vorzugsweise eines Clients, ausgegeben.

Die Fig. 2 zeigt eine bevorzugte Ausführungsform von Klassifikationsdaten KD, welche Zuordnungsdaten ZD aufweisen, welche wiederum jeweilige eindeutige Zuordnungen jeweiliger detektierter Zellbilder zu Zellklassen indizieren. Ferner weisen die Klassifikationsdaten KD Konfidenzmaßdaten KM auf, welche ein jeweiliges Konfidenzmaß für ein jeweiliges detektiertes Zellbild bezogen auf die jeweilige eindeutige Zuordnung des detektierten Zellbildes zu einer von mehreren Zellklassen indizieren.

Die Fig. 3 zeigt ein beispielhaftes Klassenbild KBX mit einer regelmäßigen räumlichen Anordnung einzelner Zellbilder Z. Ein Teilausschnitt TAY ist mittels eines gestrichelten Rechteckes dargestellt. Die Zellbilder Z sind hierbei in einer vorbestimmten Abfolge angeordnet, welche in Abhängigkeit der Konfidenzmaße der entsprechenden Zellbilder Z gewählt wird. Beispielsweise weist das Zellbild Z1 das höchste Konfidenzmaß auf, das darauf folgende Zellbild Z2 das nächst höhere Konfidenzmaß usw..

Wie zuvor ausgeführt besteht der Vorteil des vorgeschlagenen Bildverarbeitungsverfahrens darin, dass ein Pathologe die in mehreren Gesamtbildern GB1, GB2, GB3 detektierten einzelnen Zellbilder nicht in den räumlichen Anordnungen betrachten muss, wie sie z.B. in dem Gesamtbild GB11 gegeben sind, sondern dass der Pathologe in dem Teilausschnitt TAY lediglich Zellbilder aus einer bestimmten einzelnen Zellklasse des Klassenbildes KBX bzw. in dem Teilausschnitt TAX angezeigt bekommt.

Vorzugsweise erfolgen die Schritte S1 bis S4 auf einem Server und der Schritt S100 auf einem Client.

Vorzugsweise kann ein Nutzer durch eine Nutzereingabe NEX eine Zuordnung eines Zellbildes ZEX zu einer Zellklasse modifizieren und so korrigieren, vorzugsweise auf dem Client.

Für die Aufgabe des Detektierens von einzelnen Zellbildern in den Gesamtbildern mittels eines rechnergestützten Algorithmus sowie des Bestimmens der Klassifikationsdaten mittels des rechnergestützten Algorithmus eignet sich als rechnergestützter Algorithmus das Neuronale Netz nach T. Lin, P. Goyal, R. Girshick, K. He and P. Dollar, "Focal Loss for Dense Object Detection," in IEEE Transactions on Pattern Analysis and Machine Intelligence, vol. 42, no. 2, pp. 318-327, 1 Feb. 2020. Dieser Algorithmus wurde genutzt, um die hier illustrierten Beispiele zu erzeugen.

Alternative Algorithmen für neuronale Netze zur Lösung der Aufgabe des des Detektierens von einzelnen Zellbildern in den Gesamtbildern mittels eines rechnergestützten Algorithmus sowie des Bestimmens der Klassifikationsdaten mittels des rechnergestützten Algorithmus finden sich in den Veröffentlichungen
- P. Sermanet, D. Eigen, X. Zhang, M. Mathieu, R. Fergus, and Y. LeCun, "Overfeat: Integrated recognition, localization and detection using convolutional networks," arXiv:1312.6229, 2013;
- R. Girshick, J. Donahue, T. Darrell, and J. Malik, "Rich feature hierarchies for accurate object detection and semantic segmentation," in CVPR, 2014;
- S. Ren, K. He, R. Girshick, and J. Sun, "Faster r-cnn: Towards realtime object detection with region proposal networks," in NIPS, 2015, pp. 91-99.;
- J. Redmon and A. Farhadi, "Yolo9000: better, faster, stronger," arXiv:1612.08242, 2016.;
- W. Liu, D. Anguelov, D. Erhan, C. Szegedy, S. Reed, C.-Y. Fu, and A. C. Berg, "Ssd: Single shot multibox detector," in ECCV, 2016.;
- Z. Shen, Z. Liu, J. Li, Y. G. Jiang, Y. Chen, and X. Xue, "Dsod: Learning deeply supervised object detectors from scratch," in ICCV, 2017.

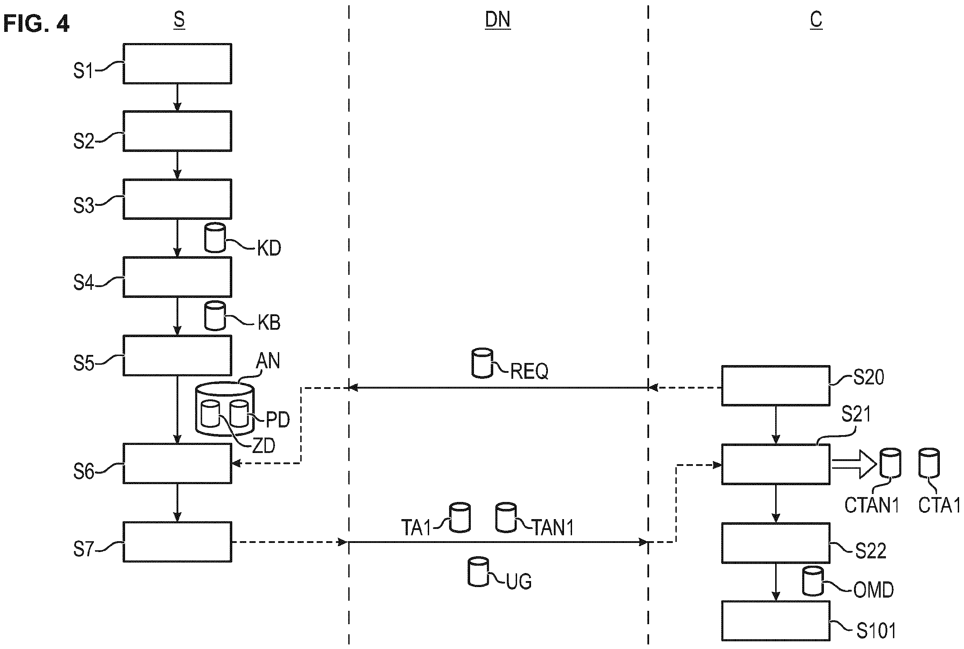

Die Fig. 4 zeigt weitere bevorzugte Schritte des vorgeschlagenen Bildverarbeitungsverfahrens gemäß einer bevorzugten Ausführungsform. Es werden zunächst die Schritte S1, S2, S3 und S4 auf einer Serverseite bzw. auf einem Server durchgeführt, wie zuvor in Bezug auf die Fig. 1 erläutert. In einem Schritt S5 erfolgt dann auf dem Server das Generieren eines Annotationsdatensatzes AN. Der Annotationsdatensatz AN weist einen Zuordnungsdatensatz ZD auf, welcher für jedes der Klassenbilder jeweilige Zuordnung der jeweiligen Zellbilder zu einer zu dem Klassenbild korrespondierenden Zellklasse indiziert. Der Annotationsdatensatz AN weist ferner Positionsdaten PD auf, welche jeweilige örtliche Positionen der jeweiligen Zellbilder innerhalb der Klassenbilder indizieren.

In einem Schritt S6 wartet der Server ab, bis eine Anforderung durch einen Client vorliegt. Der Client C fordert in einem Schritt S20 einen Teilausschnitt eines Klassenbildes an. Der Client C tut dies durch Übermitteln bzw. Übersenden einer Anforderungsnachricht bzw. eines Anforderungsdatensatzes REQ an den Server S über ein Datennetzwerk DN.

Auf Empfangen des Anforderungsdatensatzes bzw. der Anforderungsnachricht REQ hin wechselt der Server S in den Schritt S7. In dem Schritt S7 überträgt der Server S den Teilausschnitt TA1 eines Klassenbildes sowie ferner wenigstens einen zu dem Teilausschnitt TA1 korrespondierenden Teil-Annotationsdatensatz TAN1 als Teil des Annotationsdatensatzes AN an den Client C. Der Client C erhält in einem Schritt S21 den Teilausschnitt TA1 und den Teil-Annotationsdatensatz TAN1 und speichert den Teil-Annotationsdatensatz als temporären Teil-Annotationsdatensatz CTAN1 ab. Ferner speichert der Client in dem Schritt S21 vorzugsweise den Teilausschnitt TA1 als temporär gespeicherten Teilausschnitt CTA1 ab.

In einem Schritt S22 generiert der Client C optische Markierungen in Form eines optischen Markierungsdatensatzes OMD. Der optische Markierungsdatensatz OMD weist jeweilige optische Markierungen für jeweilige Zellbilder des Teilausschnittes CTA1 auf Basis wenigstens des Teil-Annotationsdatensatzes CTAN1 auf. Die jeweiligen optischen Markierungen indizieren jeweilige Zuordnung der jeweiligen Zellbilder zu einer zu dem Klassenbild korrespondierenden Zellklasse.

Vorzugsweise überträgt der Server S ein einer alternativen Ausführungsform in dem Schritt S7 nicht nur den Teil-Annotationsdatensatz TA1, welcher zu dem Teilausschnitt TA1 gehört, sondern bereits den gesamten Annotationsdatensatz AN. Hierdurch wird dann eben auch wenigstens jener Teil-Annotationsdatensatz TAN1 von dem Server an den Client übertragen, welcher zu dem übertragenen Teilausschnitt TA1 korrespondiert.

In einem Schritt S101 erfolgt dann das Anzeigen des Teilausschnittes TA1 bzw. CTA1 zusammen mit den zugehörigen optischen Markierungen auf dem Client.

Das hier beschriebene bevorzugte Ausführungsbeispiel des vorgeschlagenen Bildbearbeitungsverfahrens ist insbesondere deshalb vorteilhaft, da ein Clientsystem C durch einen Nutzer bzw. Pathologen verwendet werden kann, um eine Zuordnung von Zellbildern zu Zellklassen überprüfen zu können, ohne dass sofort sämtliche detektierten Zellbilder aller Klassenbilder auf einmal an den Client übertragen werden müssen. Eine Gesamtheit aller Klassenbilder stellt eine sehr große Datenmenge dar, deren Übertragung von einem Server an einen Client beim Datennetzwerk hin sehr viel Zeit in Anspruch nehmen kann. Möchte ein Nutzer bzw. ein Pathologe ein Clientsystem C nutzen, um Bilddaten und Klassifikationsergebnisse bzw. Zuordnungsergebnisse eines Algorithmus über ein Datennetz DN hin von einem Server S abzurufen und dann zu überprüfen, so ist das vorgeschlagene Verfahren gemäß der hier aufgezeigten Ausführungsform vorteilhaft, da lediglich zunächst nur ein Teilausschnitt TA1 eines Klassenbildes übermittelt und dann angezeigt wird. Würde die Gesamtheit der Bilddaten aller Klassenbilder ad hoc auf einmal von dem Server S über das Datennetz DN an den Client C übertragen werden, so würde dies eine große Zeitdauer benötigen und somit eine große Verzögerung bzw. Latenz für den Pathologen bei der Überprüfung der Klassenzuordnungen von Zellbildern an dem Client C bedeuten.

Es wird also ein Protokoll einer Datenverarbeitung auf einem verteilten System eines Clients und eines Servers vorgeschlagen, wobei bezogen auf einen technischen Zweck angemerkt werden kann, dass der Server verantwortlich ist zur Generierung von optischen Markierungen. Es wird also erst auf dem Client und somit dort, wo die optischen Markierungen verwendet und angezeigt werden, eine Generierung der optischen Markierung herbeigeführt, sodass der Server selber nicht für die gesamten Zellklassenbilder diese optischen Markierungen generieren muss, sondern der Client dieses eben nur für den empfangenen Teilausschnitt auf Basis des Teil-Annotationsdatensatzes durchführen muss. Es wird somit darauf abgestellt, dass Rechenressourcen zur Generierung von optischen Markierungen nur begrenzt verfügbar sind und bei dem Client nur insofern abgerufen bzw. verwendet werden, wie diese auch zum späteren Anzeigen bezogen auf den entsprechenden Teilausschnitt notwendig sind.

Die Fig. 11 zeigt, wie zuvor beschrieben, ein beispielhaftes Klassenbild KB1 einer einzelnen Zellklasse, in diesem Fall der Klasse Makrophagen.

Die Fig. 12A zeigt einen beispielhaften Teilausschnitt TA1 mit 10x8 einzelnen detektierten Zellbildern Z, welche durch gleiche optische Markierung OM1 markiert sind. Der Pathologe kann dann diesen Teilausschnitt betrachten und durch visuelle Inspektion die einzelnen Zellbilder Z hinsichtlich ihrer Klassenzuordnung überprüfen.

Vorzugsweise wird in dem Schritt S7 ferner von dem Server S über das Datennetzwerk DN an den Client C eine Übersichtsgrafik UG des Klassenbildes KB1 in verringerter Auflösung übertragen und dann auf der Clientseite in dem Schritt S101 angezeigt. Eine solche Übersichtsgrafik UG, wie in der Fig. 12B dargestellt, kann dann mittels einer Lagemarkierung L dem Nutzer bzw. dem Pathologen auch anzeigen, aus welchem Bereich des Klassenbildes KB1 der Teilausschnitt TA1 stammt. Hierdurch wird selbst bei Darstellung nur eines Teilausschnittes TA1 aus der Fig. 12A dem Nutzer in vorteilhafter Weise durch Anzeigen der Übersichtsgrafik des Klassenbildes KB1 und der Lagemarkierung L die Information übermittelt, ob die Konfidenzmaße für die Zuordnung der Zellbilder zu der entsprechenden Zellklasse eher hoch oder eher niedrig waren, da ja der Zellklasse bzw. dem Klassenbild KB1 zugeordneten Zellbilder in einer Abfolge angeordnet sind, welche in Abhängigkeit der Konfidenzmaße der Zuordnung gewählt ist.

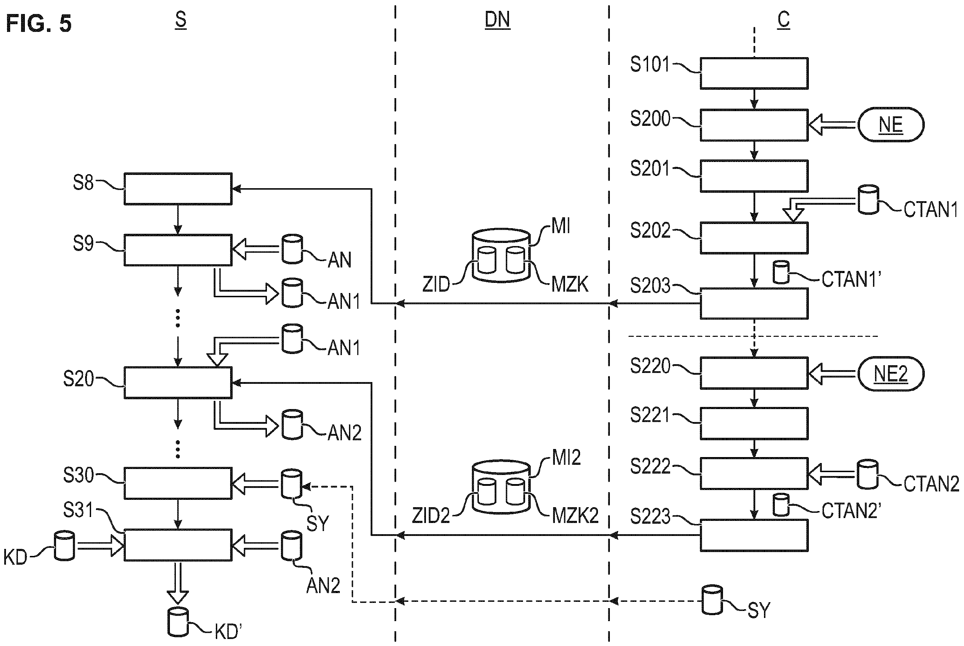

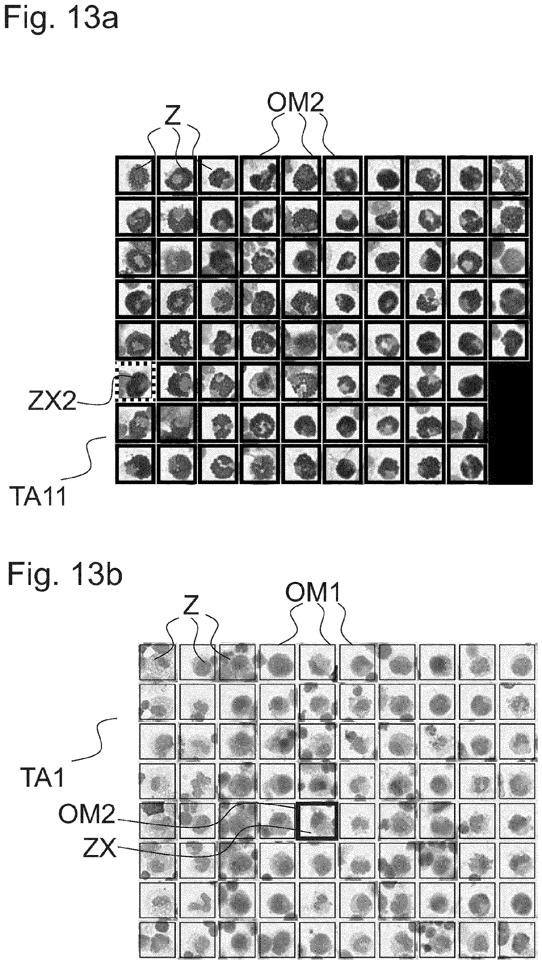

Die Fig. 5 zeigt weitere bevorzugte Schritte einer bevorzugten Ausführungsform des vorgeschlagenen Bildvearbeitungsverfahrens. Auf den Schritt des Anzeigens S101 aus der Fig. 4 hin folgt auf der Clientseite C ein Schritt S200. In dem Schritt S200 erfolgt ein Entgegennehmen einer Nutzereingabe NE, welche eine Auswahl eines angezeigten Zellbildes indiziert, vorzugsweise eines einzelnen angezeigten Zellbildes. Ferner indiziert die Nutzereingabe NE eine neue Zuordnung des ausgewählten Zellbildes zu einer modifizierten Zellklasse. Die Fig. 13B zeigt hierzu den Teilausschnitt TA1 mit den zuvor erläuterten optischen Markierungen OM1, welche eine Zuordnung der Zellen Z zu einer gleichen Zellklasse entsprechend des zugehörigen Klassenbildes des Teilausschnittes TA1 indizieren. Wählt ein Nutzer mittels einer Nutzereingabe unter Verwendung beispielsweise einer Computermaus oder einer Tastatur eine bestimmte Zelle bzw. ein bestimmtes Zellbild ZX aus, so kann der Nutzer dann ferner im Zuge der Nutzereingabe auch beispielsweise durch Eingabe einer Zahl entsprechend einer Zellklassenzahl indizieren, zu welcher Zellklasse die Zelle ZX um klassifiziert werden soll. Der Nutzer indiziert also eine modifizierte Zellklasse. Vorzugsweise kann die modifizierte Zellklasse nicht durch Eintippen einer Zahl entsprechend einer Zellklassenzahl an einer Tastatur eingegeben werden, sondern durch Anklicken einer Zellklasse KL1, KL2, KL3 aus einer Legende LG, wie in der Fig. 10B beispielhaft dargestellt, auswählen.

In der Fig. 5 findet sich ein Schritt S201, in welchem der Client für das ausgewählte Zellbild ZX korrespondierend zu der modifizierten Zellklasse dann eine neue optische Markierung OM2 generiert und auch anzeigt, wie in der Fig. 13B dargestellt. Das ausgewählte Zellbild ZX verbleibt jedoch in dem Teilausschnitt TA1 bzw. dem zugehörigen Klassenbild und wird zunächst nicht hieraus entfernt. Dieses ist vorteilhaft, da ein weiterer Pathologe später die hier vorgenommene Umklassifizierung der Zelle ZX seinerseits dann angezeigt bekommt bei Betrachten des Teilausschnittes TA1 diese Umklassifizierung überprüfen und gegebenenfalls rückgängig machen kann. Hierdurch wird also ermöglicht, dass Klassenzuordnungen einer Zelle durch einen ersten Pathologen auf dem Clientsystem später durch einen weiteren Pathologen Client überprüft und gegebenenfalls rückgängig gemacht werden können.

Hierzu modifiziert der Client den temporär abgespeicherten Teil-Annotationsdatensatz CTAN1 in einem Schritt S202, siehe Fig. 5, in Korrespondenz zu dem ausgewählten Zellbild ZX und der modifizierten Zellklasse. Es wird dann der modifizierte bzw. geänderte Teil-Annotationsdatensatz CTAN1' erstellt. In einem Schritt S203 übermittelt der Client an den Server S über das Datennetzwerk DN eine Information, welche das ausgewählte Zellbild ZX und die modifizierte Zellklasse indiziert. Hierzu übermittelt der Client C eine Modifikationsinformation MI, welche Identifikationsdaten ZID aufweisen, welche die ausgewählte Zelle bzw. das ausgewählte Zellbild ZX identifizieren bzw. indizieren. Ferner weist die Modifikationsinformation MI die modifizierte Zellklasse in Form der Klassenidentifikationsdaten MZK auf.

In einem Schritt S8 nimmt dann das Server S die Modifikationsinformation MI entgegen und geht über in den Schritt S9. In dem Schritt S9 modifiziert das Server den Annotationsdatensatz AN in Korrespondenz zu der empfangenen Information MI bzw. in Korrespondenz zu dem ausgewählten Zellbild ZX in der modifizierten Zellklasse. Der Server S generiert dann also die modifizierten Annotationsdaten AN1.

Vorgeschlagen wird also ein technisches Verfahren, bei welchem ein Nutzer durch eine ständige geführte Mensch-Maschine-Interaktion die technische Aufgabe einer Klassifikation von Zellbildern zu Zellklassen durchführen kann und hierbei durch das vorgeschlagene Ausführungsbeispiel eines Verfahren unterstützt wird. Der Nutzer muss auf dem Client lediglich ein Zellbild auswählen und eine modifizierte Zellklasse vorgeben, wobei dann automatisch nicht nur die entsprechende optische Markierung in der Anzeige des Client geändert wird, sondern eben auch eine entsprechende Information an den Server übermittelt wird, welcher den dort vorgehaltenen Annotationsdatensatz in Korrespondenz zu dem ausgewählten Zellbild und der modifizierten Zellklasse modifiziert. Es wird also der Server als zentrales System zum Nachhalten von Änderungen einer Zuordnung von Zellbildern zu Zellklassen verwendet, sodass ein zentraler Datensatz dort vorhanden ist, welcher die durch einen Nutzer an einem Client vorgenommenen Änderungen von Zuordnungen weiter nachhält. Hierzu muss nicht der gesamte Annotationsdatensatz von dem Server an den Client hin und dann auch nach einer Änderung entsprechend rückübertragen werden, sondern es wird lediglich eine Information bezogen auf das ausgewählte Zellbild und die modifizierte Zellklasse von dem Client zurück an den Server übertragen. Hierdurch wird ein Datenaufkommen bei der Übertragung über das Datennetzwerk minimiert und somit schneller gemacht als bei einer Gesamtübertragung des Annotationsdatensatzes von dem Server an den Client und auch wieder zurück.

Clientseitig können auf dem Client vorzugsweise weitere Schritte ausgeführt werden. Diese Schritte S220 bis S223 können auf dem gleichen Client ausgeführt werden oder aber zu einem späteren Zeitpunkt auf einem anderen Client. In einem Schritt S220 erfolgt analog zu dem zuvor beschriebenen Schritt S200 ein Entgegennehmen einer Nutzereingabe NE2, welche eine Auswahl eines angezeigten Zellbildes indiziert, und welche ferner eine Zuordnung des ausgewählten Zellbildes zu einer modifizierten Zellklasse indiziert. Es kann beispielsweise zu einem entsprechenden späteren Zeitpunkt ein weiterer Teilausschnitt TA11, siehe Fig. 13A, einer anderen Zellklasse bzw. eines anderen Zellbildes - oder aber der gleichen Zellklasse bzw. des gleichen Zellbildes, auf dem Client C oder einem anderen Client angezeigt werden. In einem darauffolgenden Schritt S221 analog zu dem zuvor beschriebenen Schritt S201 kann dann auf einer Clientseite eine neue optische Markierung für das ausgewählte Zellbild, beispielsweise das Zellbild ZX2 aus der Fig. 13A, generiert und angezeigt werden. In einem Schritt S222 analog zu dem zuvor beschriebenen Schritt S202 kann dann clientseitig eine Modifikation eines temporär abgespeicherten Teil-Annotationsdatensatzes CTAN2 in Korrespondenz zu dem selektierten bzw. ausgewählten Zellbild ZX2 und der modifizierten Zellklasse auf dem Client modifiziert werden, um beispielsweise den modifizierten Teil-Annotationsdatensatz CTAN2' zu erhalten. In einem Schritt S223 analog zu dem zuvor beschriebenen Schritt S203 erfolgt dann ein Übermitteln einer Modifikationsinformation MI2 von dem Client an den Server, welche mittels einer Information ZID2 das ausgewählte Zellbild ZX2 und die modifizierte Zellklasse mittels der Daten MZK2 indiziert. Auf einer Serverseite S können dann die bereits modifizierten Annotationsdaten AN1 in einem Schritt S20 analog zu dem zuvor beschriebenen Schritt S9 in Korrespondenz zu den Modifikationsdaten MI2 bzw. in Korrespondenz zu dem ausgewählten Zellbild ZX2 und der modifizierten Zellklasse modifiziert werden, um die modifizierten Annotationsdaten AN2 zu erhalten.

Das hier vorgeschlagene Ausfürungsbeispiel ist insbesondere daher vorteilhaft, da hier der Nutzer in einer geführten Mensch-Maschine-Interaktion eine Überprüfung der Zuordnung von Zellbildern zu Zellklassen, wie sie durch den computergestützten Algorithmus durchgeführt wurde, überprüfen und korrigieren kann, wobei jeweils nur ein angeforderter Teilausschnitt von dem Server an den Client übertragen wird und erst nach dem Übertragen des entsprechenden Teilausschnittes die optischen Markierungen auf dem Client für diesen zweiten Teilausschnitt generiert und angezeigt werden. Der Nutzer muss also nicht mit dem Client ein gesamtes Klassenbild empfangen, sondern kann dieses jeweils durch das Anfordern von Teilausschnitten tun. Der Nutzer kann also in einer interaktiven Suche und Abfrage gespeicherter Bilder effizienter die Klassifikationsaufgabe einer Zuordnung von Zellbildern zu Zellklassen, welche durch den computergestützten Algorithmus vorgenommen wurde, überprüfen und korrigieren.

In einem späteren Schritt S30 kann dann auf dem Server S eine Synchronisationsanforderung SY entgegengenommen werden. Diese Synchronisationsanforderung SY kann vorzugsweise über das Datennetz DN hinweg von einem Clientsystem C entgegengenommen werden. Auf Empfangen der Synchronisationsanforderung SY hin synchronisiert in einem Schritt S31 der Server die Klassifikationsdaten KD mit den vorliegenden modifizierten Annotationsdaten AN2. Hierdurch werden synchronisierte Klassifikationsdaten KD' erhalten. Der Vorteil liegt darin, dass verschiedene Nutzer an verschiedenen Clientsystemen C eine Klassifikation von Zellbildern überprüfen und modifizieren kann. Es können also mehrere Nutzer über mehrere Clientsysteme hinweg den gleichen Annotationsdatensatz AN modifizieren und es kann so eine die Modifikationsinformation in dem modifizierten Annotationsdatensatz AN2 nachgehalten werden. Erst dann, wenn der Server S eine entsprechende Synchronisationsanforderung SY erhält, wird dann die aktuell vorliegende Modifikation einer Zuordnung von Zellbildern zu Zellklassen zurück auf den Klassifikationsdatensatz KD synchronisiert, sodass ein modifizierter Klassifikationsdatensatz KD' erhalten wird. Dieser Klassifikationsdatensatz KD' kann dann verwendet werden, um geänderte optische Markierungen für Gesamtbilder, wie beispielsweise für das Gesamtbild GB11 aus der Fig. 10A, anzuzeigen.

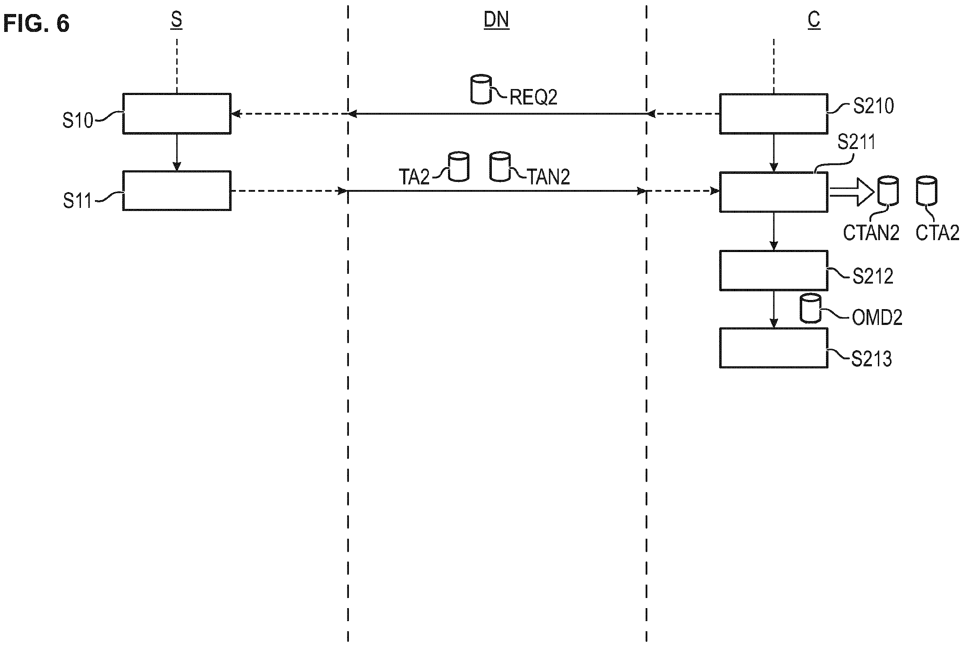

Die Fig. 6 zeigt bevorzugte Schritte zur Ausführung eines bevorzugten Ausführungsbeispiels des vorgeschlagenen Bildverarbeitungsverfahrens.

Der Server S befindet sich in einem Zustand nach dem zuvor beschriebenen Schritt S9 oder dem zuvor beschriebenen Schritt S7. In einem Schritt S10 erwartet der Server S eine Anforderungsnachricht bzw. eine Anforderungsinformation REQ. Der Client C befindet sich in einem Zustand nach dem zuvor beschriebenen Schritt S203 oder dem zuvor beschriebenen Schritt S101. In einem Schritt S210 übersendet der Client C die Anforderungsnachricht REQ an den Server S. Der erste Client C fordert also hierdurch einen zweiten Teilausschnitt des Klassenbildes an. Der Client fordert somit den zweiten Teilausschnitt des Klassenbildes von dem Server S an.

Der Server S überträgt nach Erhalt der Anforderungsnachricht REQ weitere Daten. Der Server S überträgt in einem Schritt S11 den zweiten Teilausschnitt TA2 sowie ferner wenigstens einen zu dem zweiten Teilausschnitt korrespondierenden zweiten Teil-Annotationsdatensatz TAN2. Dieses Übertragen des zweiten Teil-Annotationsdatensatzes TAN2 kann auch dadurch erfolgen, dass bei Übertragung des ersten Teil-Annotationsdatensatzes TAN1 aus Fig. 4 der gesamte Annotationsdatensatz AN übertragen wird, sodass der zweite Teil-Annotationsdatensatz TAN2 nicht zu dem gleichen Zeitpunkt von dem Server S and den Client übertragen werden muss wie zu jenem Zeitpunkt, zu welchem der zweite Teilausschnitt TA2 übertragen wird.

In einem Schritt S211 speichert der Client C temporär den zweiten Teil-Annotationsdatensatz TAN2 als temporären Datensatz CTAN2 ab. Vorzugsweise speichert der Client C in dem Schritt S211 den zweiten Teilausschnitt TA2 als temporären Teilausschnitt CTA2 ab.

In einem Schritt S212 generiert der Client C jeweilige optische Markierungen für jeweilige Zellbilder des zweiten Teilausschnittes TA2 bzw. CTA2, wobei die jeweiligen optischen Markierungen jeweilige Zuordnung der jeweiligen Zellbilder zu einer zu dem Klassenbild korrespondierenden Zellklasse indizieren. Die optischen Markierungen sind dann durch die optischen Markierungsdaten OMD2 gegeben. In einem Schritt S213 zeigt der Client den zweiten Teilausschnitt TA2 bzw. CTA2 sowie die zugehörigen optischen Markierungen OMD2 an. Das hier beschriebene Ausführungsbeispiel ist vorteilhaft, da der Client C nicht nur zu einem ersten Zeitpunkt einen ersten Teilausschnitt TA1, wie in Fig. 4 dargestellt, anfordern und dann anzeigen kann, sondern dass der Client C auch zu einem weiteren Zeitpunkt dann einen zweiten Teilausschnitt TA2 des gleichen Klassenbildes zu einem späteren Zeitpunkt anfordern kann. Es muss also ein Klassenbild einer bestimmten Klasse nicht vollständig von dem Server S an den Client C übertragen werden, um zumindest einen Teilausschnitt aus diesem Klassenbild auf dem Server S anzuzeigen. Der Client C kann sukzessive Teilausschnitte von dem Server S über das Datennetzwerk DN hin empfangen, sodass sich eine geringere Latenz der Bilddatenübertragung ergibt als bei vollständiger Übersendung eines gesamten Klassenbildes oder aber aller Klassenbilder. Ferner kann der Client C sukzessive sequenziell zu unterschiedlichen Zeitpunkten entsprechende optische Markierungsdaten OMD bzw. OMD2 generieren und muss diese optischen Markierungsdaten nicht ad hoc für ein gesamtes Klassenbild generieren. Auch hierdurch ergibt sich eine geringere Latenz für das Generieren und das Anzeigen der optischen Markierungsdaten eines bestimmten Teilausschnittes.

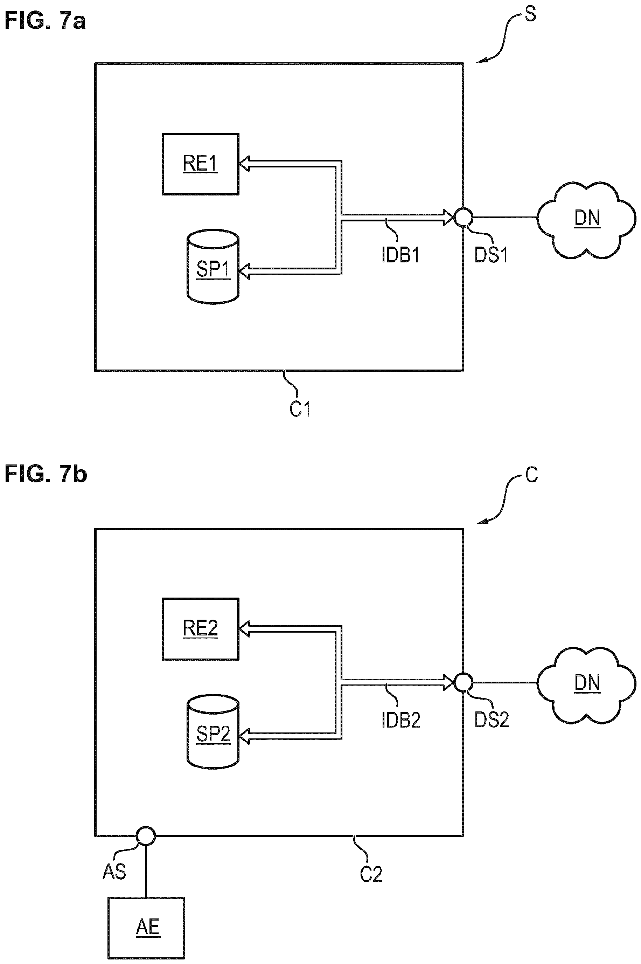

Die Fig. 7A zeigt einen beispielhaften Server S. Der Server weist vorzugsweise eine Datenschnittstelle DS1 zu einem Datennetzwerk DN auf. Der Server S weist ferner eine Recheneinheit RE1 auf. Ferner weist der Server eine Speichereinheit SP1 auf. Der Server ist vorzugsweise ein Computer bzw. Rechner C1. Die Datenschnittstelle DS1 sowie die Recheneinheit RE1 und die Speichereinheit SP1 sind vorzugsweise über einen internen Datenbus IDB1 miteinander verbunden.

Fig. 7B zeigt einen beispielhaften Client C. Der Client C weist eine Datenschnittstelle DS2 zu einem Datennetzwerk DN hin auf. Der Client C weist ferner eine Recheneinheit RE2 auf, sowie eine Speichereinheit SP2. Die Datenschnittstelle DS2, die Recheneinheit RE2 sowie die Speichereinheit SP2 sind vorzugsweise über einen internen Datenbus IDB2 miteinander verbunden. Der Client C ist vorzugsweise ein Computer bzw. Rechner C2. Der Client C weist vorzugsweise eine Ausgabeschnittstelle AS hin zu einer Anzeigeeinheit AE auf. Vorzugsweise ist die Anzeigeeinheit AE integraler Bestandteil des Clients C.

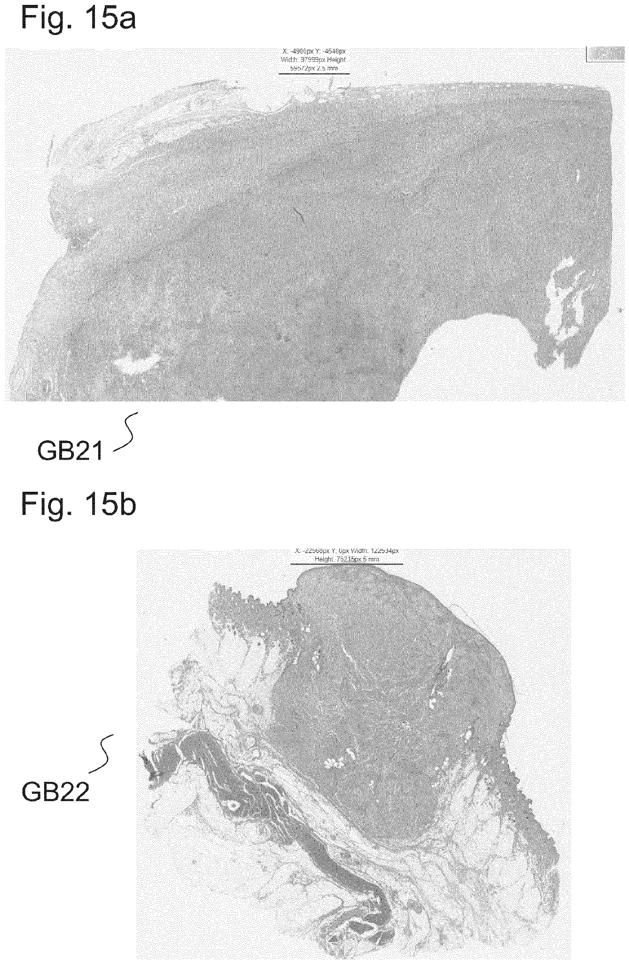

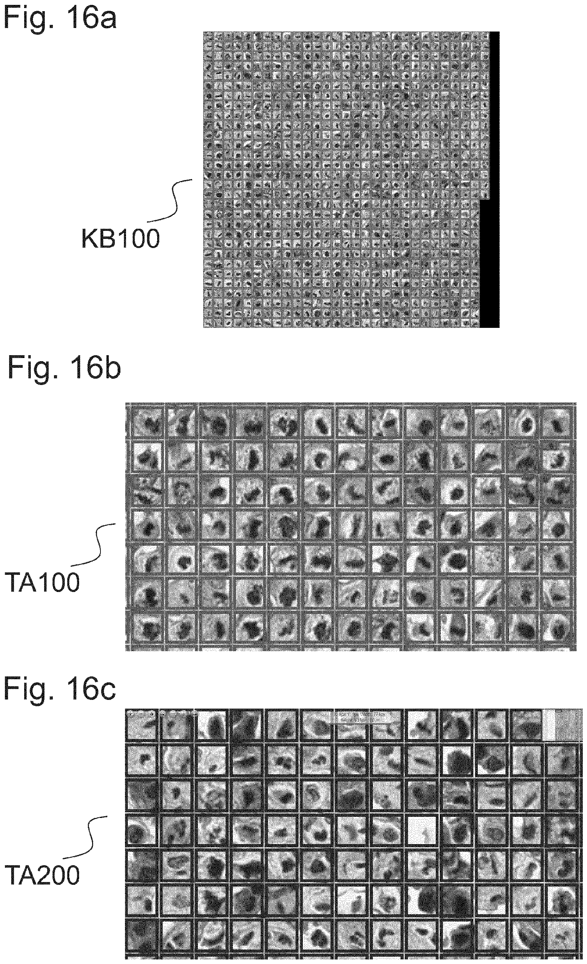

Die zuvor gemachten Erläuterung wurden unter Bezug auf Gesamtbilder ausgeführt, welche jeweils eine Patientenzellprobe in Form eines Zellausstrichs einer flüssigen Patientenprobe repräsentieren, siehe insbesondere Figuren 8, 9, 10, 11, 12 und 13. Für den Fall, dass die Gesamtbilder jeweils eine Patientengewebeprobe in Form eines Gewebeschnittes repräsentieren, zeigen die Figuren 15a und 15b jeweilige Gesamtbilder GB21 bzw. GB22. Die Figur 16a zeigt ein beispielhaftes Klassenbild KB100 für die Zellklasse Mitosezelle. Die Figur 16b zeigt hierzu einen beispielhaften Teilausschnitt TA100 des Klassenbildes KB100 mit Zellen der Zellklasse Mitosezellen. Figur 16c zeigt einen beispielhaften Teilausschnitt TA200 für die Zellklasse von Nicht-Mitosezellen. Es wird für den Fachmann durch Betrachtung der Figuren 15 und 16 deutlich, dass die vorgeschlagene Erfindung und ihre Ausführungsbeispiele bzw. Ausführungsformen somit auch für Gesamtbilder ausgeführt werden können, welche jeweils eine Patientengewebeprobe in Form eines Gewebeschnittes repräsentieren.

Offenbart wird hierin auch ein Bildverarbeitungsverfahren zur Durchführung auf einem Server S, welches verschiedene Schritte aufweist: Bereitstellen von mehreren Gesamtbildern GB1, GB2, GB3, Detektieren von einzelnen Zellbildern Z in den Gesamtbildern GB1, GB2, GB3 mittels eines rechnergestützten Algorithmus, Bestimmen von Klassifikationsdaten KD mittels des rechnergestützten Algorithmus, wobei die Klassifikationsdaten KD eine jeweilige eindeutige Zuordnung eines jeweiligen detektierten Zellbildes Z zu einer jeweiligen Zellklasse KL1, KL2, KL3 indizieren, und wobei die Klassifikationsdaten KD ferner ein jeweiliges Konfidenzmaß bezogen auf die jeweilige eindeutige Zuordnung aufweisen, Generieren von jeweiligen Klassenbildern KB1 für die jeweiligen Zellklassen KL1, wobei ein Klassenbild KB1 einer Zellklasse KL1 die der Zellklasse zugeordneten Zellbilder in einer regelmäßigen Anordnung und mit einer vorbestimmten Abfolge der zugeordneten Zellbilder in Abhängigkeit der Konfidenzmaße der zugeordneten Zellbilder gewählt wird, Generieren eines Annotationsdatensatzes AN, welcher für jedes der Klassenbilder jeweilige Zuordnungen der jeweiligen Zellbilder zu einer zu dem Klassenbild korrespondierenden Zellklasse indiziert und welcher ferner jeweilige örtliche Positionen der jeweiligen Zellbilder innerhalb der Klassenbilder indiziert, Übertragen eines Teilausschnittes TA1 wenigstens eines Klassenbildes KB1 sowie ferner wenigstens eines zu dem Teilausschnitt TA1 korrespondierenden Teil-Annotationsdatensatzes TAN1 an einen Client C, Entgegennehmen einer Information von dem Client C, welche ein ausgewähltes Zellbild ZX und eine modifizierte Zellklasse indiziert, Modifizieren des Annotationsdatensatzes AN in Korrespondenz zu dem ausgewählten Zellbild ZX und der modifizierten Zellklasse.
Vorzugsweise erfolgt auf dem Server S ein Entgegennehmen einer Synchronisationsanforderung SY und ein Synchronisieren der Klassifikationsdaten KD mit dem modifizierten Annotationsdatensatz AN1, AN2.

Offenbart wird hierin ferner ein Bildverarbeitungsverfahren zur Durchführung auf einem Client C, aufweisend verschiedene Schritte: Anfordern eines Teilausschnittes TA1 wenigstens eines Klassenbildes KB1 von einem Server S, wobei ein Klassenbild zu einer Zellklasse zugeordnete Zellbilder in einer regelmäßigen Anordnung und mit einer vorbestimmten Abfolge widergibt, Empfangen des Teilausschnittes TA1 des Klassenbildes sowie ferner wenigstens eines zu dem Teilausschnitt TA1 korrespondierenden Teil-Annotationsdatensatzes TAN1 von dem Server S und temporäres Abspeichern des Teil-Annotationsdatensatzes TAN1, wobei der Teil-Annotationsdatensatz TAN1 für den Teilausschnitt TA1 des Klassenbildes jeweilige Zuordnungen jeweiliger Zellbilder Z zu einer zu dem Klassenbild korrespondierenden Zellklasse indiziert und wobei der Teil-Annotationsdatensatz TAN1 jeweilige örtliche Positionen der jeweiligen Zellbilder Z innerhalb des Klassenbildes indiziert, Generieren von jeweiligen optischen Markierungen OM1 für jeweilige Zellbilder des Teilausschnittes auf Basis wenigstens des Teil-Annotationsdatensatzes TAN1, wobei die jeweiligen optischen Markierungen OM1 jeweilige Zuordnungen der jeweiligen Zellbilder Z zu einer zu dem Klassenbild korrespondierenden Zellklasse indizieren, sowie Anzeigen des Teilausschnittes TA1 sowie zugehöriger optischer Markierungen OM1 von Zellbildern des Teilausschnittes TA1. Vorzugsweise weist das Bildverarbeitungsverfahren zur Durchführung auf einem Client C ferner die Schritte auf: Entgegennehmen einer Nutzereingabe NE, welche eine Auswahl eines angezeigten Zellbildes ZX indiziert und welche ferner eine neue Zuordnung des ausgewählten Zellbildes ZX zu einer modifizierten Zellklasse indiziert, Generieren und Anzeigen einer neuen optischen Markierung OM2 für das ausgewählte Zellbild ZX korrespondierend zu der modifizierten zugeordneten Zellklasse, Modifizieren des temporär abgespeicherten Teil-Annotationsdatensatzes CTAN1 in Korrespondenz zu dem ausgewählten Zellbild ZX und der modifizierten Zellklasse, Übermitteln einer Information MI an den Server, welche das ausgewählte Zellbild ZX und die modifizierte Zellklasse indiziert.

Voreschlagen wird ferner ein Computerprogrammprodukt, umfassend Befehle, die bei der Ausführung des Programms durch einen Computer in Form eines Servers diesen veranlassen, das Bildverarbeitungsverfahren zur Durchführung auf dem Server S durchzuführen.

Voreschlagen wird ferner ein Computerprogrammprodukt, umfassend Befehle, die bei der Ausführung des Programms durch einen Computer in Form eines Clients diesen veranlassen, das Bildverarbeitungsverfahren zur Durchführung auf dem Client C durchzuführen.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine hierin beschriebene Recheneinheit kann auch als eine Hardwarekomponente in Form eines Prozessors aufgefasst werden. Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Ein Computerprogrammprodukt kann auf einem digitalen Speichermedium bereitgestellt werden und kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

## Patentansprüche

1. Bildverarbeitungsverfahren zum Anzeigen von Zellen (Z) mehrerer Gesamtbilder (GB1, GB2, GB3),
wobei ein jeweiliges Gesamtbild (GB1, GB2, GB3) eine jeweilige Patientengewebeprobe oder eine jeweilige Patientenzellprobe repräsentiert,
aufweisend die Schritte, vorzugsweise auf einem Server (S),
- Bereitstellen der Gesamtbilder (GB1, GB2, GB3),
- Detektieren von einzelnen Zellbildern (Z) in den Gesamtbildern (GB1, GB2, GB3) mittels eines rechnergestützten Algorithmus,
- Bestimmen von Klassifikationsdaten (KD) mittels des rechnergestützten Algorithmus,
wobei die Klassifikationsdaten (KD) eine jeweilige eindeutige Zuordnung eines jeweiligen detektierten Zellbildes (Z) zu einer von mehreren Zellklassen (KL1, KL2, KL3) indizieren,
und wobei die Klassifikationsdaten (KD) ferner ein jeweiliges Konfidenzmaß bezogen auf die jeweilige eindeutige Zuordnung aufweisen,
- Generieren von jeweiligen Klassenbildern (KB1) für die jeweiligen Zellklassen (KL1),
wobei ein Klassenbild (KB1) einer Zellklasse (KL1) die der Zellklasse zugeordneten Zellbilder in einer regelmäßigen Anordnung und mit einer vorbestimmten Abfolge widergibt
und wobei ferner die Abfolge der zugeordneten Zellbilder (Z) in Abhängigkeit der Konfidenzmaße der zugeordneten Zellbilder gewählt wird,
sowie ferner, vorzugsweise auf einem Client (C),
- Anzeigen eines Teilausschnittes (TA1) wenigstens eines Klassenbildes (KB1).

2. Verfahren nach Anspruch 1,
wobei die zuvor genannten Schritte des Bereitstellens, des Detektierens, des Bestimmens und des Generierens auf einem Server (S) erfolgen,
ferner aufweisend
- Generieren, auf dem Server (S), eines Annotationsdatensatzes (AN), welcher für jedes der Klassenbilder (KB1) jeweilige Zuordnungen der jeweiligen Zellbilder (Z) zu einer zu dem Klassenbild (KB1) korrespondierenden Zellklasse indiziert
und welcher ferner jeweilige örtliche Positionen der jeweiligen Zellbilder (Z) innerhalb der Klassenbilder indiziert,
- Anfordern eines Teilausschnittes (TA1) eines Klassenbildes durch einen Client (C),
- Übertragen des Teilausschnittes (TA1) sowie ferner wenigstens eines zu dem Teilausschnitt (TA1) korrespondierenden Teil-Annotationsdatensatzes (TAN1) von dem Server (S) an den Client (C) und temporäres Abspeichern des Teil-Annotationsdatensatzes (TAN1) auf dem Client (C),
- Generieren, auf dem Client (C), von jeweiligen optischen Markierungen (OM) für jeweilige Zellbilder des Teilausschnittes auf Basis wenigstens des Teil-Annotationsdatensatzes, wobei die jeweiligen optischen Markierungen (OM1) jeweilige Zuordnungen der jeweiligen Zellbilder (Z) zu einer zu dem Klassenbild korrespondierenden Zellklasse indizieren,
- Anzeigen des Teilausschnittes (TA1) sowie der zugehörigen optischen Markierungen (OM1) auf dem Client (C) auf einer Anzeigeeinheit (AE).

3. Verfahren nach Anspruch 2,
- Entgegennehmen, auf dem Client (C), einer Nutzereingabe (NE), welche eine Auswahl eines angezeigten Zellbildes (ZX) indiziert und welche ferner eine neue Zuordnung des ausgewählten Zellbildes (ZX) zu einer modifizierten Zellklasse (KL2) indiziert,
- Generieren und Anzeigen, auf dem Client (C), einer neuen optischen Markierung (OM2) für das ausgewählte Zellbild (ZX) korrespondierend zu der modifizierten Zellklasse (KL2),
- Modifizieren des temporär abgespeicherten Teil-Annotationsdatensatzes (CTAN1) in Korrespondenz zu dem ausgewählten Zellbild (ZX) und der modifizierten Zellklasse (KL2) auf dem Client (C),
- Übermitteln einer Information von dem Client (C) an den Server (S), welche das ausgewählte Zellbild (ZX) und die modifizierte Zellklasse (KL2) indiziert,
- Modifizieren des Annotationsdatensatzes (AN) auf dem Server (S) in Korrespondenz zu dem ausgewählten Zellbild (ZX) und der modifizierten Zellklasse (KL2).

4. Verfahren nach Anspruch 2 oder 3,
wobei der Teilausschnitt (TA1) ein erster Teilausschnitt ist,
ferner aufweisend
- Anfordern eines zweiten Teilausschnittes (TA2) des Klassenbildes durch den Client (C),
- Übertragen des zweiten Teilausschnittes (TA2) sowie ferner wenigstens eines zu dem zweiten Teilausschnitt (TA2) korrespondierenden zweiten Teil-Annotationsdatensatzes (TAN2) von dem Server (S) an den Client (C) und temporäres Abspeichern des zweiten Teil-Annotationsdatensatzes (TAN2) auf dem Client (C),
- Generieren, auf dem Client (C), von jeweiligen optischen Markierungen (OM2) für jeweilige Zellbilder des zweiten Teilausschnittes (TA2) auf Basis wenigstens des zweiten Teil-Annotationsdatensatzes (TAN2), wobei die jeweiligen optischen Markierungen jeweilige Zuordnungen der jeweiligen Zellbilder zu einer zu dem Klassenbild korrespondierenden Zellklasse indizieren,
- Anzeigen des zweiten Teilausschnittes (TA2) sowie zugehöriger optischen Markierungen (OM2) auf dem Client (C).

5. Verfahren nach Anspruch 3,
ferner aufweisend, auf dem Server (S),
- Entgegennehmen einer Synchronisationsanforderung (SY),
- Synchronisieren der Klassifikationsdaten (KD) mit dem modifizierten Annotationsdatensatz (AN2).

6. Bildverarbeitungsverfahren zur Durchführung auf einem Server (S),
aufweisend die Schritte
- Bereitstellen von mehreren Gesamtbildern (GB1, GB2, GB3), wobei ein jeweiliges Gesamtbild eine jeweilige Patientengewebeprobe oder eine jeweilige Patientenzellprobe repräsentiert,
- Detektieren von einzelnen Zellbildern (Z) in den Gesamtbildern (GB1, GB2, GB3) mittels eines rechnergestützten Algorithmus,
- Bestimmen von Klassifikationsdaten (KD) mittels des rechnergestützten Algorithmus,
wobei die Klassifikationsdaten (KD) eine jeweilige eindeutige Zuordnung eines jeweiligen detektierten Zellbildes (Z) zu einer jeweiligen Zellklasse (KL1, KL2, KL3) indizieren,
und wobei die Klassifikationsdaten (KD) ferner ein jeweiliges Konfidenzmaß bezogen auf die jeweilige eindeutige Zuordnung aufweisen,
- Generieren von jeweiligen Klassenbildern (KB1) für die jeweiligen Zellklassen,
wobei ein Klassenbild einer Zellklasse die der Zellklasse zugeordneten Zellbilder in einer regelmäßigen Anordnung und mit einer vorbestimmten Abfolge der zugeordneten Zellbilder in Abhängigkeit der Konfidenzmaße der zugeordneten Zellbilder gewählt wird,
- Generieren eines Annotationsdatensatzes (AN),
welcher für jedes der Klassenbilder jeweilige Zuordnungen der jeweiligen Zellbilder zu einer zu dem Klassenbild korrespondierenden Zellklasse indiziert
und welcher ferner jeweilige örtliche Positionen der jeweiligen Zellbilder innerhalb der Klassenbilder indiziert,
- Übertragen eines Teilausschnittes (TA1) wenigstens eines Klassenbildes (KB1) sowie ferner wenigstens eines zu dem Teilausschnitt (TA1) korrespondierenden Teil-Annotationsdatensatzes an einen Client (C),
- Entgegennehmen einer Information (MI) von dem Client (C), welche ein ausgewähltes Zellbild (ZX) und eine modifizierte Zellklasse (KL2) indiziert,
- Modifizieren des Annotationsdatensatzes (AN) in Korrespondenz zu dem ausgewählten Zellbild (ZX) und der modifizierten Zellklasse (KL2).

7. Bildverarbeitungsverfahren nach Anspruch 6,
ferner aufweisend
- Entgegennehmen einer Synchronisationsanforderung (SY),
- Synchronisieren der Klassifikationsdaten (KD) mit dem modifizierten Annotationsdatensatz (AN2).

8. Bildverarbeitungsverfahren zur Durchführung auf einem Client (C),
aufweisend
- Anfordern eines Teilausschnittes (TA1) wenigstens eines Klassenbildes (KB1) von einem Server (S), wobei ein Klassenbild zu einer Zellklasse zugeordnete Zellbilder in einer regelmäßigen Anordnung und mit einer vorbestimmten Abfolge widergibt,
- Empfangen des Teilausschnittes (TA1) des Klassenbildes sowie ferner wenigstens eines zu dem Teilausschnitt (TA1) korrespondierenden Teil-Annotationsdatensatzes (TAN1) von dem Server (S) und temporäres Abspeichern des Teil-Annotationsdatensatzes,
wobei der Teil-Annotationsdatensatz (TAN1) für den Teilausschnitt (TA1) des Klassenbildes jeweilige Zuordnungen jeweiliger Zellbilder (Z) zu einer zu dem Klassenbild korrespondierenden Zellklasse (KL1) indiziert und wobei der Teil-Annotationsdatensatz jeweilige örtliche Positionen der jeweiligen Zellbilder innerhalb des Klassenbildes indiziert,
- Generieren von jeweiligen optischen Markierungen (OM1) für jeweilige Zellbilder (Z) des Teilausschnittes (TA1) auf Basis wenigstens des Teil-Annotationsdatensatzes (TAN1), wobei die jeweiligen optischen Markierungen jeweilige Zuordnungen der jeweiligen Zellbilder zu einer zu dem Klassenbild korrespondierenden Zellklasse indizieren,
- Anzeigen des Teilausschnittes (TA1) sowie zugehöriger optischer Markierungen (OM1) von Zellbildern des Teilausschnittes.

9. Bildverarbeitungsverfahren nach Anspruch 8,
ferner aufweisend
- Entgegennehmen einer Nutzereingabe (NE), welche eine Auswahl eines angezeigten Zellbildes (ZX) indiziert und welche ferner eine neue Zuordnung des ausgewählten Zellbildes (ZX) zu einer modifizierten Zellklasse (KL2) indiziert,
- Generieren und Anzeigen einer neuen optischen Markierung (OM2) für das ausgewählte Zellbild (ZX) korrespondierend zu der modifizierten zugeordneten Zellklasse,
- Modifizieren des temporär abgespeicherten Teil-Annotationsdatensatzes (CTAN1') in Korrespondenz zu dem ausgewählten Zellbild und der modifizierten Zellklasse,
- Übermitteln einer Information (MI) an das Server (S), welche das ausgewählte Zellbild (ZX) und die modifizierte Zellklasse (KL2) indiziert.

10. Computerprogrammprodukt,
umfassend Befehle, die bei der Ausführung des Programms durch einen Computer in Form eines Servers diesen veranlassen, das Verfahren nach Anspruch 6 durchzuführen.

11. Computerprogrammprodukt
umfassend Befehle, die bei der Ausführung des Programms durch einen Computer in Form eines Clients diesen veranlassen, das Verfahren nach Anspruch 8 durchzuführen.

## Geänderte Patentansprüche

### Geänderte Patentansprüche gemäss Regel 137(2) EPÜ.

1. Bildverarbeitungsverfahren zum Anzeigen von Zellen (Z) mehrerer Gesamtbilder (GB1, GB2, GB3),
wobei ein jeweiliges Gesamtbild (GB1, GB2, GB3) eine jeweilige Patientengewebeprobe oder eine jeweilige Patientenzellprobe repräsentiert,
aufweisend die Schritte, auf einem Server (S),
- Bereitstellen der Gesamtbilder (GB1, GB2, GB3),
- Detektieren von einzelnen Zellbildern (Z) in den Gesamtbildern (GB1, GB2, GB3) mittels eines rechnergestützten Algorithmus,
- Bestimmen von Klassifikationsdaten (KD) mittels des rechnergestützten Algorithmus,
wobei die Klassifikationsdaten (KD) eine jeweilige eindeutige Zuordnung eines jeweiligen detektierten Zellbildes (Z) zu einer von mehreren Zellklassen (KL1, KL2, KL3) indizieren,
und wobei die Klassifikationsdaten (KD) ferner ein jeweiliges Konfidenzmaß bezogen auf die jeweilige eindeutige Zuordnung aufweisen,
- Generieren von jeweiligen Klassenbildern (KB1) für die jeweiligen Zellklassen (KL1),
wobei ein Klassenbild (KB1) einer Zellklasse (KL1) die der Zellklasse zugeordneten Zellbilder in einer regelmäßigen Anordnung und mit einer vorbestimmten Abfolge widergibt
und wobei ferner die Abfolge der zugeordneten Zellbilder (Z) in Abhängigkeit der Konfidenzmaße der zugeordneten Zellbilder gewählt wird,
**gekennzeichnet durch**, auf einem Client (C),
- Anzeigen eines Teilausschnittes (TA1) wenigstens eines Klassenbildes (KB1), wobei der Teilausschnitt mehrere Zellbilder aus den mehreren Gesamtbildern einer bestimmten Zellklasse aufweist.

2. Verfahren nach Anspruch 1,
ferner aufweisend
- Generieren, auf dem Server (S), eines Annotationsdatensatzes (AN), welcher für jedes der Klassenbilder (KB1) jeweilige Zuordnungen der jeweiligen Zellbilder (Z) zu einer zu dem Klassenbild (KB1) korrespondierenden Zellklasse indiziert und welcher ferner jeweilige örtliche Positionen der jeweiligen Zellbilder (Z) innerhalb der Klassenbilder indiziert,
- Anfordern eines Teilausschnittes (TA1) eines Klassenbildes durch einen Client (C),
- Übertragen des Teilausschnittes (TA1) sowie ferner wenigstens eines zu dem Teilausschnitt (TA1) korrespondierenden Teil-Annotationsdatensatzes (TAN1) von dem Server (S) an den Client (C) und temporäres Abspeichern des Teil-Annotationsdatensatzes (TAN1) auf dem Client (C),
- Generieren, auf dem Client (C), von jeweiligen optischen Markierungen (OM) für jeweilige Zellbilder des Teilausschnittes auf Basis wenigstens des Teil-Annotationsdatensatzes, wobei die jeweiligen optischen Markierungen (OM1) jeweilige Zuordnungen der jeweiligen Zellbilder (Z) zu einer zu dem Klassenbild korrespondierenden Zellklasse indizieren,
- Anzeigen des Teilausschnittes (TA1) sowie der zugehörigen optischen Markierungen (OM1) auf dem Client (C) auf einer Anzeigeeinheit (AE).

3. Verfahren nach Anspruch 2,
- Entgegennehmen, auf dem Client (C), einer Nutzereingabe (NE), welche eine Auswahl eines angezeigten Zellbildes (ZX) indiziert und welche ferner eine neue Zuordnung des ausgewählten Zellbildes (ZX) zu einer modifizierten Zellklasse (KL2) indiziert,
- Generieren und Anzeigen, auf dem Client (C), einer neuen optischen Markierung (OM2) für das ausgewählte Zellbild (ZX) korrespondierend zu der modifizierten Zellklasse (KL2),
- Modifizieren des temporär abgespeicherten Teil-Annotationsdatensatzes (CTAN1) in Korrespondenz zu dem ausgewählten Zellbild (ZX) und der modifizierten Zellklasse (KL2) auf dem Client (C),
- Übermitteln einer Information von dem Client (C) an den Server (S), welche das ausgewählte Zellbild (ZX) und die modifizierte Zellklasse (KL2) indiziert,
- Modifizieren des Annotationsdatensatzes (AN) auf dem Server (S) in Korrespondenz zu dem ausgewählten Zellbild (ZX) und der modifizierten Zellklasse (KL2).

4. Verfahren nach Anspruch 2 oder 3,
wobei der Teilausschnitt (TA1) ein erster Teilausschnitt ist,
ferner aufweisend
- Anfordern eines zweiten Teilausschnittes (TA2) des Klassenbildes durch den Client (C),
- Übertragen des zweiten Teilausschnittes (TA2) sowie ferner wenigstens eines zu dem zweiten Teilausschnitt (TA2) korrespondierenden zweiten Teil-Annotationsdatensatzes (TAN2) von dem Server (S) an den Client (C) und temporäres Abspeichern des zweiten Teil-Annotationsdatensatzes (TAN2) auf dem Client (C),
- Generieren, auf dem Client (C), von jeweiligen optischen Markierungen (OM2) für jeweilige Zellbilder des zweiten Teilausschnittes (TA2) auf Basis wenigstens des zweiten Teil-Annotationsdatensatzes (TAN2), wobei die jeweiligen optischen Markierungen jeweilige Zuordnungen der jeweiligen Zellbilder zu einer zu dem Klassenbild korrespondierenden Zellklasse indizieren,
- Anzeigen des zweiten Teilausschnittes (TA2) sowie zugehöriger optischen Markierungen (OM2) auf dem Client (C).

5. Verfahren nach Anspruch 3,
ferner aufweisend, auf dem Server (S),
- Entgegennehmen einer Synchronisationsanforderung (SY),
- Synchronisieren der Klassifikationsdaten (KD) mit dem modifizierten Annotationsdatensatz (AN2).

6. Bildverarbeitungsverfahren zur Durchführung auf einem Server (S),
aufweisend die Schritte
- Bereitstellen von mehreren Gesamtbildern (GB1, GB2, GB3), wobei ein jeweiliges Gesamtbild eine jeweilige Patientengewebeprobe oder eine jeweilige Patientenzellprobe repräsentiert,
- Detektieren von einzelnen Zellbildern (Z) in den Gesamtbildern (GB1, GB2, GB3) mittels eines rechnergestützten Algorithmus,
- Bestimmen von Klassifikationsdaten (KD) mittels des rechnergestützten Algorithmus,
wobei die Klassifikationsdaten (KD) eine jeweilige eindeutige Zuordnung eines jeweiligen detektierten Zellbildes (Z) zu einer jeweiligen Zellklasse (KL1, KL2, KL3) indizieren,
und wobei die Klassifikationsdaten (KD) ferner ein jeweiliges Konfidenzmaß bezogen auf die jeweilige eindeutige Zuordnung aufweisen,
- Generieren von jeweiligen Klassenbildern (KB1) für die jeweiligen Zellklassen,
wobei ein Klassenbild einer Zellklasse die der Zellklasse zugeordneten Zellbilder in einer regelmäßigen Anordnung und mit einer vorbestimmten Abfolge der zugeordneten Zellbilder in Abhängigkeit der Konfidenzmaße der zugeordneten Zellbilder gewählt wird,
- Generieren eines Annotationsdatensatzes (AN),
welcher für jedes der Klassenbilder jeweilige Zuordnungen der jeweiligen Zellbilder zu einer zu dem Klassenbild korrespondierenden Zellklasse indiziert
und welcher ferner jeweilige örtliche Positionen der jeweiligen Zellbilder innerhalb der Klassenbilder indiziert,
- Übertragen eines Teilausschnittes (TA1) wenigstens eines Klassenbildes (KB1) sowie ferner wenigstens eines zu dem Teilausschnitt (TA1) korrespondierenden Teil-Annotationsdatensatzes an einen Client (C), wobei der Teilausschnitt mehrere Zellbilder aus den mehreren Gesamtbildern einer bestimmten Zellklasse aufweist,
- Entgegennehmen einer Information (MI) von dem Client (C), welche ein ausgewähltes Zellbild (ZX) und eine modifizierte Zellklasse (KL2) indiziert,
- Modifizieren des Annotationsdatensatzes (AN) in Korrespondenz zu dem ausgewählten Zellbild (ZX) und der modifizierten Zellklasse (KL2).

7. Bildverarbeitungsverfahren nach Anspruch 6,
ferner aufweisend
- Entgegennehmen einer Synchronisationsanforderung (SY),
- Synchronisieren der Klassifikationsdaten (KD) mit dem modifizierten Annotationsdatensatz (AN2).

8. Bildverarbeitungsverfahren zur Durchführung auf einem Client (C),
aufweisend
- Anfordern eines Teilausschnittes (TA1) wenigstens eines Klassenbildes (KB1) von einem Server (S), wobei ein Klassenbild zu einer Zellklasse zugeordnete Zellbilder in einer regelmäßigen Anordnung und mit einer vorbestimmten Abfolge widergibt,
- Empfangen des Teilausschnittes (TA1) des Klassenbildes sowie ferner wenigstens eines zu dem Teilausschnitt (TA1) korrespondierenden Teil-Annotationsdatensatzes (TAN1) von dem Server (S) und temporäres Abspeichern des Teil-Annotationsdatensatzes, wobei der Teilausschnitt mehrere Zellbilder aus den mehreren Gesamtbildern einer bestimmten Zellklasse aufweist,
wobei der Teil-Annotationsdatensatz (TAN1) für den Teilausschnitt (TA1) des Klassenbildes jeweilige Zuordnungen jeweiliger Zellbilder (Z) zu einer zu dem Klassenbild korrespondierenden Zellklasse (KL1) indiziert und wobei der Teil-Annotationsdatensatz jeweilige örtliche Positionen der jeweiligen Zellbilder innerhalb des Klassenbildes indiziert,
- Generieren von jeweiligen optischen Markierungen (OM1) für jeweilige Zellbilder (Z) des Teilausschnittes (TA1) auf Basis wenigstens des Teil-Annotationsdatensatzes (TAN1), wobei die jeweiligen optischen Markierungen jeweilige Zuordnungen der jeweiligen Zellbilder zu einer zu dem Klassenbild korrespondierenden Zellklasse indizieren,
- Anzeigen des Teilausschnittes (TA1) sowie zugehöriger optischer Markierungen (OM1) von Zellbildern des Teilausschnittes.

9. Bildverarbeitungsverfahren nach Anspruch 8,
ferner aufweisend
- Entgegennehmen einer Nutzereingabe (NE), welche eine Auswahl eines angezeigten Zellbildes (ZX) indiziert und welche ferner eine neue Zuordnung des ausgewählten Zellbildes (ZX) zu einer modifizierten Zellklasse (KL2) indiziert,
- Generieren und Anzeigen einer neuen optischen Markierung (OM2) für das ausgewählte Zellbild (ZX) korrespondierend zu der modifizierten zugeordneten Zellklasse,
- Modifizieren des temporär abgespeicherten Teil-Annotationsdatensatzes (CTAN1') in Korrespondenz zu dem ausgewählten Zellbild und der modifizierten Zellklasse,
- Übermitteln einer Information (MI) an das Server (S), welche das ausgewählte Zellbild (ZX) und die modifizierte Zellklasse (KL2) indiziert.

10. Computerprogrammprodukt,
umfassend Befehle, die bei der Ausführung des Programms durch einen Computer in Form eines Servers diesen veranlassen, das Verfahren nach Anspruch 6 durchzuführen.

11. Computerprogrammprodukt
umfassend Befehle, die bei der Ausführung des Programms durch einen Computer in Form eines Clients diesen veranlassen, das Verfahren nach Anspruch 8 durchzuführen.
